(19) Europäisches Patentamt
European Patent Office
Office européen des brevets

(11) EP 3 974 027 B1

(12) **EUROPEAN PATENT SPECIFICATION**

(45) Date of publication and mention of the grant of the patent:
**23.10.2024 Bulletin 2024/43**

(21) Application number: **20805414.8**

(22) Date of filing: **14.05.2020**

(51) International Patent Classification (IPC):
***C07K 14/575*** (2006.01) ***A61P 9/00*** (2006.01)
***A61P 25/00*** (2006.01) ***A61P 27/02*** (2006.01)
***A61P 29/00*** (2006.01) *A61K 38/00* (2006.01)

(52) Cooperative Patent Classification (CPC):
**A61P 9/00; A61P 25/00; A61P 27/02; A61P 29/00; C07K 14/575;** A61K 38/00

(86) International application number:
**PCT/JP2020/019344**

(87) International publication number:
**WO 2020/230867 (19.11.2020 Gazette 2020/47)**

# (54) STABILIZED PACAP PEPTIDE

STABILISIERTES PACAP-PEPTID

PEPTIDE PACAP STABILISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.05.2019 JP 2019091696**

(43) Date of publication of application:
**30.03.2022 Bulletin 2022/13**

(73) Proprietor: **Senju Pharmaceutical Co., Ltd.**
**Osaka-shi,**
**Osaka 541-0048 (JP)**

(72) Inventors:
- **MACHIDA, Shinnosuke**
  **Osaka-shi, Osaka 541-0048 (JP)**
- **NAKAJIMA, Takeshi**
  **Osaka-shi, Osaka 541-0048 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 752 158** **WO-A1-2005/102375**
**JP-A- 2004 168 697**

- **DOAN NGOC-DUC ET AL: "Design and in vitro characterization of PAC1/VPAC1-selective agonists with potent neuroprotective effects", BIOCHEMICAL PHARMACOLOGY, vol. 81, no. 4, 27 November 2010 (2010-11-27), US, pages 552 - 561, XP093059058, ISSN: 0006-2952, DOI: 10.1016/j.bcp.2010.11.015**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

TECHNICAL FIELD

The present invention relates to a stabilized peptide having a physiological activity of PACAP, and to a neuroprotective agent, a vascular endothelial function ameliorator, a corneal neurite formation promoter, a lacrimal secretagogue, a dry-eye treatment agent, a corneal epithelial or corneal endothelial damage treatment agent, a neurotrophic keratitis treatment agent, an anti-inflammatory agent or a pharmaceutical composition, which includes the peptide.

BACKGROUND ART

Neurons are cells constituting the nervous system, which is largely divided into the central nervous system and the peripheral nervous system. Neurons are susceptible to damage by external factors, including cerebrovascular accidents such as stroke and cerebral infarction, and internal factors including accumulation of abnormal proteins, oxidative stress and inflammation, while regenerative ability is also low, and therefore when damaged they can result in notable impairment of patient QOL. Neurodegenerative diseases associated with neurodegeneration and loss of the central nervous system include neurodegenerative diseases such as Alzheimer's disease, Parkinson's disease, Huntington's disease, amyotrophic lateral sclerosis and multiple sclerosis, and optic neurodegenerative diseases such as glaucoma or optic neuropathy, and also sensory neurodegenerative diseases such as nerve deafness.

Advances in neuroscience have led to the discovery of numerous neuroprotective factors, and they are expected for developing as a preventive or therapeutic agent for nervous disorder. Drugs that decrease free radicals or excitatory amino acids responsible for neurodegeneration, and drugs that are able to protect and/or repair neurons (for example, immunophilin ligands, such as neurotrophic factors, or for immunosuppressive agents), have been found to exhibit neuroprotective action. Endogenous proteins such as pituitary adenylate cyclase-activating polypeptide (PACAP), CD44 and human brain carboxypeptidase B (HBCPB) have also been shown to exhibit neuroprotective action (PTLs 1 and 2).

Pituitary adenylate cyclase-activating polypeptide (PACAP) is a neuropeptide originally discovered in sheep hypothalamus extract. PACAP can exhibit activity which stimulates cAMP formation in anterior pituitary cells. PACAP includes PACAP38 which consists of 38 amino acid residues, and PACAP27 which consists of 27 amino acid residues, both of which have equivalent activity (NPLs 1 and 2). PACAP belongs to the vasoactive intestinal polypeptide (VIP)/secretin/glucagon superfamily, and the sequence of human PACAP27 has 68% identity with vasoactive intestinal polypeptide (VIP). PACAP and VIP both bind to PAC1 receptor (PAC1R), VPAC1 receptor (VPAC1R) and VPAC2 receptor (VPAC2R), but they differ in their affinities for their respective receptors. PAC1R binds to PACAP with high selectivity. The affinity of PAC1R for PACAP is more than 1000 times higher than the affinity of PAC1R for VIP. VPAC1R and VPAC2R, on the other hand, both have equal affinity for PACAP and VIP. PACAP has a variety of physiological effects, being known to have physiological activity as a neuroprotective substance, immunosuppressive factor, vasodilating factor, exocrine secretagogue (PTL 3) and neurite formation promoting factor (PTL 4).

Medical drug is being developed utilizing the various physiological activities of PACAP. However, most peptides such as PACAP are unstable in aqueous solution and are associated with the problem of a short half-life due to lack of protease resistance.

[CITATION LIST]

[PATENT LITERATURE]

[PTL 1] Japanese Patent Public Inspection No. 2014-510101
[PTL 2] Japanese Unexamined Patent Publication No. 2012-232952
[PTL 3] Japanese Unexamined Patent Publication No. 2009-269818
[PTL 4] International Patent Publication No. WO2005/102375, pamphlet

[NON PATENT LITERATURE]

[NPL 1] S. Bourgault (2009) Current Medicinal Chemistry 16, 4462-4480
[NPL 2] Louise Dickson (2009) Pharmacology & Therapeutics, 12, 294-316
[NPL3] Ngoc-Duc Doan et al. (2011) refers to the design and in vitro characterization of PAC1/VPAC1-selective

agonists with potent neuroprotective effects SUMMARY OF INVENTION

[TECHNICAL PROBLEM]

The present invention is intended to provide a stabilized PACAP peptide with high stability in aqueous solution, as well as a neuroprotective agent, a neurite formation promoter, a lacrimal gland secretagogue, a therapeutic or prophylactic pharmaceutical composition for dry-eye, a therapeutic or prophylactic pharmaceutical composition for corneal epithelial or corneal endothelial damage, a therapeutic or prophylactic pharmaceutical composition for neuropathy, or a therapeutic or prophylactic composition for neurotrophic keratitis, which includes the peptide.

[SOLUTION TO PROBLEM]

The present inventors have conducted avid research for the purpose of increasing the stability of PACAP in aqueous solution, and have completed this invention upon finding that it is possible to vastly increase its stability in aqueous solution by independently replacing each of the aspartic acids at position 3 and position 8 of PACAP peptide with a residue selected from the group consisting of tetrazole-substituted aspartic acid (Tz), tetrazole-substituted glutamic acid (egTz), tetrazole-substituted homoglutamic acid (nvTz), sulfoxy-substituted aspartic acid (cya) and oxadiazolone-substituted aspartic acid (SOxa).

The invention is set out in the claims.

[ADVANTAGEOUS EFFECTS OF INVENTION]

According to the invention it is possible to greatly stabilize PACAP, which has been unstable in aqueous solution.

DESCRIPTION OF EMBODIMENTS

The present invention relates to a peptide as claimed consisting of a sequence in which the aspartic acid residues at position 3 and position 8 of PACAP are each independently replaced by a residue selected from the group consisting of tetrazole-substituted aspartic acid (Tz), tetrazole-substituted glutamic acid (egTz), tetrazole-substituted homoglutamic acid (nvTz), sulfoxy-substituted aspartic acid (cya) and oxadiazolone-substituted aspartic acid (SOxa), or its partially modified sequence, wherein the peptide has an affinity for PAC1R, VPAC1R and/or VPAC2R equivalent to that of PACAP. The "equivalent affinity" means that the EC50 value of the peptide for each receptor is up to 100 times that of PACAP. The PACAP may be PACAP38 consisting of 38 residues, or PACAP27 consisting of 27 residues. PACAP38 and PACAP27 have the following sequences:

PACAP38:HSDGIFTDSYSRYRKQMAVKKYLAAVLGKRYKQRVKNK (SEQ ID NO: 1)
PACAP27:HSDGIFTDSYSRYRKQMAVKKYLAAVL (SEQ ID NO: 2)

According to the invention, tetrazole-substituted aspartic acid (Tz), tetrazole-substituted glutamic acid (egTz) and tetrazole-substituted homoglutamic acid (nvTz) are non-natural amino acid residues having a structure in which the carboxyl group on the side chain of aspartic acid, glutamic acid or homoglutamic acid, respectively, is replaced with the carboxyl group isostere tetrazole. These are represented by the following formulas:

[Chemical Formula 1]

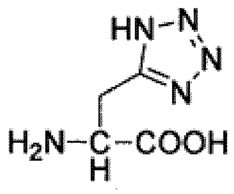

Tetrazole-substituted aspartic acid (Tz)

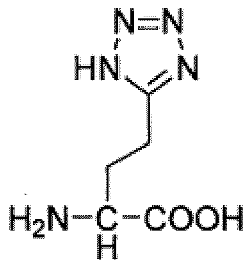

; Tetrazole-substituted glutamic acid (egTz)

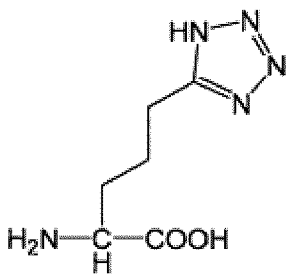

Tetrazole-substituted homoglutamic acid (nvTz)

According to the invention, sulfoxy-substituted aspartic acid (cya) and oxadiazolone-substituted aspartic acid (SOxa) are residues having a structure in which the carboxyl group on the side chain of aspartic acid is replaced with the carboxyl group isostere sulfoxy or 1,2,4-oxadiazol-5-one (oxadiazolone), respectively. These are represented by the following formulas:

[Chemical Formula 2]

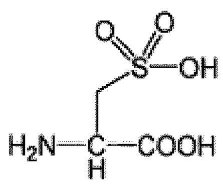

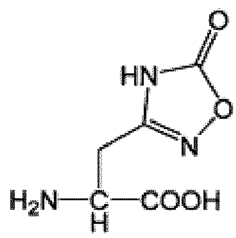

Sulfoxy-substituted aspartic acid (cya) ; Oxadiazolone-substituted aspartic acid (5Oxa)

Non-natural amino acid residues with aspartic acid at position 3 and position 8 replaced may be the L-forms, D-forms or racemic forms, so long as they have the equivalent level of physiological activity as PACAP. In particular, a peptide substituted with the L-form tetrazole-substituted aspartic acid and a peptide substituted with the D-form tetrazole-substituted aspartic acid have the equivalent level of affinity as PAC1R, VPAC1R and/or VPAC2R. With no intention to be limited to any particular theory, it is possible that including a D-form amino acid is less likely to be degraded by enzymes, allowing the stability to be increased. Non-natural amino acids other than protein-constituting amino acids may also be introduced into peptides together with protecting groups by methods such as the Fmoc method, similar to protein-constituting amino acids.

According to the invention, a "sequence which is partially modified" is a sequence in which one or more amino acids of the original sequence have been substituted, deleted or added. More preferably, a "sequence which is partially modified" is a sequence in which one or several amino acids of the original sequence have been substituted, deleted or added.

An amino acid substitution may be at any position so long as the affinity of the peptide consisting of the sequence which is partially modified, for PAC1R, VPAC1R and/or VPAC2R, does not change. From the viewpoint of maintaining affinity, the amino acid substitution in the peptide consisting of the partially modified sequence may be at position 2, position 5, position 7, position 9, position 11, position 16 and position 17 of PACAP27. According to a particularly preferred aspect, the amino acids at positions $X_1$ to $X_7$ in the following sequence: H-$X_1$-D-G-$X_2$-F-$X_3$-D-$X_4$-Y-$X_5$-R-Y-R-K-$X_6$-$X_7$-A-V-K-K-Y-L-A-A-V-L (SEQ ID NO: 3) may be substituted.

One aspect of the invention, therefore, relates to a peptide consisting of the sequence represented by the following formula:

H-$X_1$-D-G-$X_2$-F-$X_3$-D-$X_4$-Y-$X_5$-R-Y-R-K-$X_6$-$X_7$-A-V-K-K-Y-L-A-A-V-L (SEQ ID NO: 3) {wherein
$X_1$ to $X_6$ are neutral amino acids, and
$X_7$ is a non-polar amino acid},
with the aspartic acids at position 3 and position 8 each independently replaced with a residue selected from the group consisting of tetrazole-substituted aspartic acid (Tz), tetrazole-substituted glutamic acid (egTz), tetrazole-substituted homoglutamic acid (nvTz), sulfoxy-substituted aspartic acid (cya) and oxadiazolone-substituted aspartic acid (SOxa), or its modified sequence, wherein the peptide has affinity for PAC1R, VPAC1R and/or VPAC2R.

The substitution may also be conservative substitution with an amino acid having the same attributes. Examples of conservative substitutions include substitutions of amino acids within the following groups:

(i) Non-polar amino acids: Val, Leu, Ile, Met, Phe, Trp, Pro, Nle, Ala
(ii) Neutral amino acids: Ala, Ser, Thr, Tyr, Cys, Asn, Gln, Gly
(iii) Basic amino acids: Lys, Arg, His
(iv) Acidic amino acids: Asp, Glu

Among neutral amino acids, α-aminoisobutyric acid (Ai) having a methyl group substituted at the α-position of alanine and β-cyanoalanine (Cn) having the hydrogen atom at the β-position of alanine substituted with a cyano group, may be used instead of alanine. In particular, α-aminoisobutyric acid (Ai) and β-cyanoalanine (Cn) may be used as neutral amino acids at position 9 and position 11. The amino acids α-aminoisobutyric acid (Ai) and β-cyanoalanine (Cn) have the following structures:

[Chemical Formula 3]

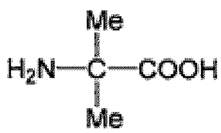
α-Aminoisobutyric acid (Ai) ;

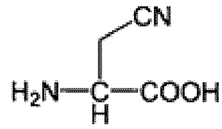
β-Cyanoalanine (Cn)

In the sequence represented by SEQ ID NO: 3 of the invention, the amino acids represented by $X_1$ to $X_7$ are preferably the following:

The neutral amino acid of $X_1$ is, in particular, alanine or serine.
The neutral amino acid of $X_2$ is, in particular, isoleucine or alanine.
The neutral amino acid of $X_3$ is, in particular, alanine or threonine.
The neutral amino acids of $X_4$ and $X_5$ are, in particular, alanine, serine, α-aminoisobutyric acid or β-cyanoalanine.
The neutral amino acid of $X_6$ is, in particular, glutamine or alanine.
The non-polar amino acid of $X_7$ is, in particular, methionine, leucine, norleucine or alanine.

According to the invention, a "modified sequence" is a sequence in which one or more amino acids of the original sequence have been substituted, deleted or added. More preferably, a "sequence which is partially modified" is a sequence in which one or several amino acids of the original sequence have been substituted, deleted or added.

The amino acid substitution may include substitution of one or more amino acids, but from the viewpoint of maintaining an activity, any number from 1 to 10, such as 1, 2, 3, 4, 5, 6, 7, 8, 9 or 10, amino acids may be substituted. A substitution of 1 to 4 amino acids is particularly preferred, a substitution of 3 amino acids is more preferred, a substitution of two amino acids is even more preferred and a substitution of one amino acid is most preferred.

An amino acid deletion may be at any position so long as the affinity of the peptide consisting of the sequence which is partially modified, for PAC1R, VPAC1R and/or VPAC2R, does not change. The number of amino acids deleted is selected as any number from 1 to 10, such as 1, 2, 3, 4, 5, 6, 7, 8, 9 or 10. From the viewpoint of not altering affinity, a deletion of one or two amino acids, and especially a deletion of one amino acid, is particularly preferred. The amino acid deletion may be a deletion at the N-terminal end or C-terminal end of the original sequence, or a deletion within the sequence. Since PACAP27 and PACAP38 have equivalent binding affinity for PAC1R, VPAC1R and/or VPAC2R, it is believed that deletion of amino acids present at the C-terminal end of PACAP38 have little effect on the affinity.

An amino acid addition may be at any position so long as the affinity of the peptide consisting of the sequence which is partially modified, for PAC1R, VPAC1R and/or VPAC2R, does not change. The number of amino acids added is selected as any number from 1 to 10, such as 1, 2, 3, 4, 5, 6, 7, 8, 9 or 10. The amino acids may be added at the N-terminal end or C-terminal end of the original sequence, or they may be added within the sequence. Since PACAP27 and PACAP38 have equivalent binding affinity for PAC1R, VPAC1R and/or VPAC2R, it is believed that addition of any amino acids at the C-terminus of PACAP27 has little effect on the affinity.

A modified sequence of the sequence listed as SEQ ID NO: 3 wherein the aspartic acid residues at position 3 and position 8 are each independently replaced by a residue selected from the group consisting of tetrazole-substituted

aspartic acid (Tz), tetrazole-substituted glutamic acid (egTz), tetrazole-substituted homoglutamic acid (nvTz), sulfoxy-substituted aspartic acid (cya) and oxadiazolone-substituted aspartic acid (SOxa), is preferably the amino acid sequence listed as SEQ ID NO: 3 with a deletion or addition of one or more amino acids. More preferably, one or two amino acids may be deleted from the amino acid sequence listed as SEQ ID NO: 3, or the following C-terminal added sequence may be added to the amino acid sequence listed as SEQ ID NO: 3. The amino acid sequence listed as SEQ ID NO: 3 may also have an addition of 1 to 3 amino acids (such as methionine) at the N-terminus.

It is not our intention to be limited to any particular theory, but since PACAP27 and PACAP38 have equivalent binding affinity for PAC1R, VPAC1R and/or VPAC2R, addition of any amino acids at the C-terminal end of PACAP27 does not effect the binding affinity for PAC1R. Therefore, the peptide with a modified sequence according to the invention may include a C-terminal added sequence at the C-terminus of the amino acid sequence listed as SEQ ID NO: 3, similar to PACAP27. A C-terminal added sequence is a sequence comprising 1 to 11 arbitrary amino acids. The C-terminal added sequence preferably corresponds to the amino acids at position 28 to position 38 of PACAP38. The following sequences may therefore be mentioned as C-terminal sequences:

GKRYKQRVKNK (SEQ ID NO: 43);
GKRYKQRVKN (SEQ ID NO: 44);
GKRYKQRVK (SEQ ID NO: 45);
GKRYKQRV (SEQ ID NO: 46);
GKRYKQR (SEQ ID NO: 47);
GKRYKQ (SEQ ID NO: 48);
GKRYK (SEQ ID NO: 49);
GKRY (SEQ ID NO: 50)
GKR;
GRR;
GK; and
GR;
G.

The peptide of the invention may use either the D-form or L-form of amino acids, or the racemic forms of amino acids, so long as affinity for PAC1R, VPAC1R and/or VPAC2R is not lost. The peptide of the invention may also comprise non-natural amino acids such as 2-aminoisobutyric acid, and may include derivatives with optional modification of N-terminal amino groups, C-terminal carboxyl groups or amino acid side chain functional groups. Examples of modifications include addition of protecting groups on the amino group (for example, acylation (formylation, acetylation, propionylation, butyrylation or isobutyrylation), mesylation, ureation, carbamation, Boc-protection or Fmoc-protection), and esterification (such as ethylation) of the carboxyl group. It may also include modification that can commonly take place in the body, such as phosphorylation, amidation, methylation, esterification and acetylation, as well as modification that takes place during the synthesis process or that is used to facilitate purification, such as biotinylation. For the purpose of extending the *in vivo* half-life of the peptide, modification such as PEGylation may also be added. From the viewpoint of increasing the stability, in particular, the free amino group of the N-terminal amino acid may be protected with a protecting group (such as an acyl group). For example, the free amino groups of N-terminal amino acids may be acylated (for example, acetylated, propionylated, butyrylated or isobutyrylated), or mesylated. Similarly, the free carboxyl groups of C-terminal amino acids may be protected by protecting groups (such as amide or ester groups). For example, the free carboxyl groups of C-terminal amino acids may be amidated or esterified. Acetylation or mesylation of the N-terminus of a peptide of the invention further increases the stability. The C-terminus may be a carboxyl group (-COOH), carboxylate (-COO-), amide (-$CONH_2$) or ester (-COOR). The peptide of the invention may also have a glycosyl chain addition (see WO2017/027848, for example).

According to one specific aspect, the invention relates to the peptides having the sequences listed in Table 1 below, or their modified sequences as claimed:

[Table 1]

**Table 1**

| | N-terminus | Sequence | C-terminus | SEQ ID No. |
|---|---|---|---|---|
| Peptide 1 | H | HSTzGI FATzAY SRYRK QMAVK KYLAA VL | NH2 | 4 |
| Peptide 2 | H | HSTzGI FATzSY ARYRK QMAVK KYLAA VL | NH2 | 5 |
| Peptide 3 | H | HSTzGI FATzAY ARYRK QMAVK KYLAA VL | NH2 | 6 |
| Peptide 4 | H | HSTzGI FATzAiY SRYRK QMAVK KYLAA VL | NH2 | 7 |
| Peptide 5 | H | HSTzGI FATzSY AiRYRK QMAVK KYLAA VL | NH2 | 8 |
| Peptide 6 | H | HSTzGI FATzAiY AiRYRK QMAVK KYLAA VL | NH2 | 9 |
| Peptide 7 | H | HSTzGI FTTzAiY AiRYRK QMAVK KYLAA VL | NH2 | 10 |
| Peptide 8 | H | HSTzGI FTTzSY SRYRK QMAVK KYLA | NH2 | 11 |
| Peptide 9 | H | HSTzGI FTTzSY SRYRK QMAVK KYLAA V | NH2 | 12 |
| Peptide 10 | H | HScyaGI FTcyaSY SRYRK QLAVK KYLAA VL | NH2 | 13 |
| Peptide 11 | H | HScyaGI FTcyaSY SRYRK QNIAVK KYLAA VL | NH2 | 14 |
| Peptide 12 | H | MHSTzGI FTTzSY SRYRK CLAVK KYLAA VL | NH2 | 15 |
| Peptide 13 | H | HSTz(D)GI FTTz(D)SY SRYRK ONIAVK KYLAA VL | NH2 | 16 |
| Peptide 14 | H | HSTzGI FTT$_Z$(D)SY SRYRK ONIAVK KYLAA VL | NH2 | 17 |
| Peptide 15 | H | HSTz(D)GI FTTzSY SRYRK ONIAVK KYLAA VL | NH2 | 18 |
| Peptide 16 | H | HSTzGI FTegTzSY SRYRK QNIAVK KYLAA VL | NH2 | 19 |
| Peptide 17 | $CH_3CH_2CO$ | HSTzGI FTTzSY SRYRK QNIAVK KYLAA VL | NH2 | 20 |
| Peptide 18 | $CH_3CH_2CH_2CO$ | HSTzGI FTTzSY SRYRK ONIAVK KYLAA VL | NH2 | 21 |
| Peptide 19 | $(CH_3)_2CHCO$ | HSTzGI FTTzSY SRYRK QNIAVK KYLAA VL | NH2 | 22 |
| Peptide 20 | $CH_3CH_2CH_2CH_aCO$ | HSTzGI FTTzSY SRYRK QNIAVK KYLAA VL | NH2 | 23 |
| Peptide 21 | $(CH_3)_2CHCH_2CO$ | HSTzG1 FTTzSY SRYRK ONIAVK KYLAA VL | NH2 | 24 |
| Peptide 22 | Ac | HS(50xa)GI FT(50xa)SY SRYRK ONIAVK KYLAA VL | NH2 | 25 |

(continued)

| | N-terminus | Sequence | C-terminus | SEQ ID No. |
|---|---|---|---|---|
| Peptide 23 | Ac | HSTzGA FTTzSY SRYRK GLAVK KYLAA VL | NH2 | 26 |
| Peptide 24 | Ac | HSTzGI FATzSY SRYRK QLAVK KYLAA VL | NH2 | 27 |
| Peptide 25 | Ac | HATzGI FATzSY SRYRK QLAVK KYLAA VL | NH2 | 28 |
| Peptide 26 | Ac | HATzGI FTTzSY SRYRK ALAVK KYLAA VL | NH2 | 29 |
| Peptide 27 | Ac | HSTzGI FATzAY SRYRK QLAVK KYLAA VL | NH2 | 30 |
| Peptide 28 | Ac | HSTzGI FATzSY SRYRK ALAVK KYLAA VL | NH2 | 31 |
| Peptide 29 | Ac | HSTzGI FATzSY SRYRK QAAVK KYLAA VL | NH2 | 32 |
| Peptide 30 | Ac | HATzGI FATzAY SRYRK AAAVK KYLAA VL | NH2 | 33 |
| Synthetic Example 1 | H | HSTzGI FATzSY CnRYRK QMAVK KYLAA VL | NH2 | 34 |
| Synthetic Example 2 | H | HSTzGI FTTzCnY CnRYRK QMAVK KYLAA VL | NH2 | 35 |
| Synthetic Example 3 | H | HSegTzGI FTTzSY SRYRK QNIAVK KYLAA VL | NH2 | 36 |
| Synthetic Example 4 | H | HSegTzGI FTegTzSY SRYRK QNIAVK KYLAA VL | NH2 | 37 |
| Synthetic Example 5 | H | HSnvTzGI FTTzSY SRYRK QNIAVK KYLAA VL | NH2 | 38 |
| Synthetic Example 6 | H | HSTzGI FTnvTzSY SRYRK QNIAVK KYLAA VL | NH2 | 39 |
| Synthetic Example 7 | H | HSnvTzGI FTnvTzSY SRYRK QNIAVK KYLAA VL | NH2 | 40 |
| Synthetic Example 8 | H | HSegTzGI FTnvTzSY SRYRK QNIAVK KYLAA VL | NH2 | 41 |
| Synthetic Example 9 | H | HSnvTzGI FTegTzSY SRYRK QNIAVK KYLAA VL | NH2 | 42 |

In the table, Tz represents tetrazole-substituted aspartic acid, egTz represents tetrazole-substituted glutamic acid, nvTz represents tetrazole-substituted homoglutamic acid, cya represents sulfoxy-substituted aspartic acid, (5Oxa) represents oxadiazolone-substituted aspartic acid, Ai represents $\alpha$-aminoisobutyric acid and Cn represents $\beta$-cyanoalanine. Tz(D) represents that tetrazole-substituted aspartic acid is the D-form.

The peptide of the invention can be produced by any production method. For example, it can be produced by solid phase synthesis or liquid phase synthesis using the Boc or Fmoc method. It can also be produced by other methods, using a method of transferring nucleic acid coding for the peptide of the invention into a gene, introducing the gene into host cells and synthesizing the peptide in the host cells. Purification after expression can be easily accomplished by a design in which a tag peptide such as a polyhistidine tag is added at the ends of the peptide.

The peptide of the invention also includes its pharmaceutically acceptable salts. Pharmaceutically acceptable salts include salts of inorganic acids (for example, hydrochlorides, hydrobromides, sulfates and phosphates), salts of

organic acids (for example, methanesulfonates, benzenesulfonates, p-toluenesulfonates, formates, acetates, trifluoroacetates, oxalates, citrates, malonates, fumarates, maleates, tartrates, succinates and malates), or salts with bases (for example, ammonium salts, methylpyridinium salts and acetylpyridinium salts). The peptide of the invention also includes hydrates or solvates.

An Fmoc-N protected amino acid wherein the side chain carboxyl group of an amino acid (aspartic acid) has been replaced by the carboxyl group isostere tetrazole can be produced by the method represented by Reaction Scheme 1, or a similar method, using Fmoc-N protected asparagine as the starting substance.

[Chemical Formula 4]

Reaction Scheme 1

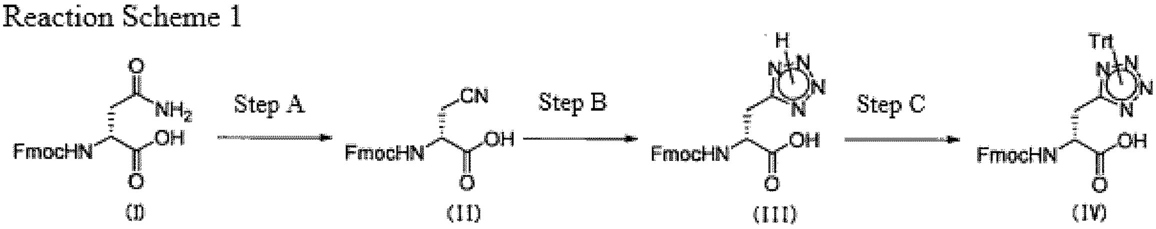


{wherein

Trt represents a trityl group, and
H or Trt group is substituting at N1 or N2 of the tetrazole}

In the method of Reaction Scheme 1, the compound represented by (I) (for example, asparagine with the amino group protected by Fmoc) is reacted with EDCI in the presence of a base in step A, to produce compound (II). In step A, the base is used at 1 to 50 equivalents and preferably 10 to 25 equivalents with respect to compound (I). The base used may be pyridine, 4-dimethylaminopyridine, triethylamine, N,N-diisopropylethylamine or N-methylmorpholine, with pyridine being preferred. EDCI is used at 1 to 10 equivalents and preferably 1 or 2 equivalents. The solvent is not particularly restricted so long as it does not affect the reaction, and examples include N,N-dimethylformamide, N,N-dimethyl acetamide, toluene, dichloromethane, tetrahydrofuran and acetonitrile, preferably acetonitrile. The reaction temperature will usually be -10 to 60°C and is preferably 0 to 30°C, while the reaction time is 0.5 to 12 hours and preferably 1 to 5 hours.

In step B, compound (II) is reacted with trimethylsilyl azide and dibutyltin oxide in toluene to produce compound (III). Trimethylsilyl azide is used at 1 to 5 equivalents and preferably 2 to 4 equivalents with respect to compound (II). Dibutyltin oxide is used at 0.3 to 1 equivalent and preferably 0.7 to 0.95 equivalent with respect to compound (II). The solvent is not particularly restricted so long as it does not affect the reaction, and examples include benzene, toluene and xylene, preferably toluene. The reaction time will usually be 0.5 to 24 hours and is preferably 0.5 to 2 hours. The reaction temperature will usually be 80 to 150°C and is preferably 80 to 100°C. The heating method may be with an oil bath or microwave, with microwave heating being preferred.

In the subsequent step C, compound (IV) can be produced by reacting trityl chloride in the presence of a base. The base used in step C may be pyridine, 4-dimethylaminopyridine, triethylamine, N,N-diisopropylethylamine or N-methylmorpholine, with N-methylmorpholine being preferred. The amount of base used is 1 to 3 equivalents and preferably 1 to 1.5 equivalents with respect to compound (IX). Trityl chloride is used at 1 to 3 equivalents and preferably 1.05 to 1.1 equivalents. The solvent is not particularly restricted so long as it does not affect the reaction, and examples include N,N-dimethylformamide, N,N-dimethyl acetamide, toluene, dichloromethane, tetrahydrofuran and acetonitrile, preferably tetrahydrofuran. The reaction temperature will usually be -10 to 60°C and is preferably 0 to 30°C. The reaction time is 0.5 to 12 hours and preferably 1 to 2 hours.

Fmoc-nvTz(Trt)-OH, having tetrazole bonded at the α-position of the amino acid via an alkyl having 3 carbon atoms, can be produced by the method represented by Reaction Scheme 2 or a similar method.

[Chemical Formula 5]

Reaction Scheme 2

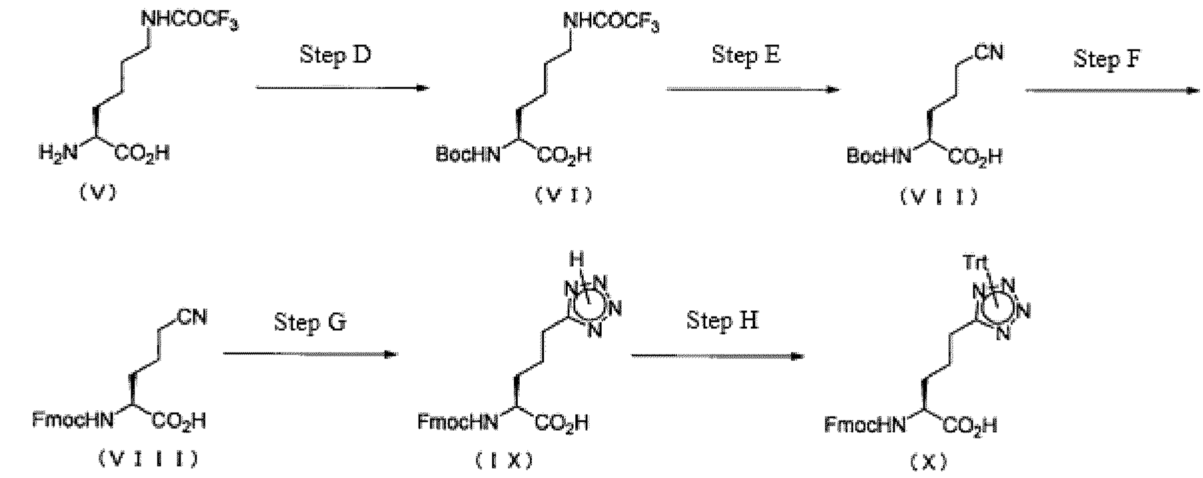

(V) (VI) (VII) (VIII) (IX) (X)

In step D of the method of Reaction Scheme 2, a compound represented by general formula for compound (V) is reacted with $Boc_2O$ in the presence of a base to produce compound (VI). The base is used at 1 to 10 equivalents and preferably 1 to 2 equivalents with respect to compound (V). The base used may be triethylamine, N,N-diisopropylamine, N-methylmorpholine, sodium hydroxide, potassium carbonate or sodium hydrogencarbonate, preferably sodium hydroxide. The $Boc_2O$ is used at 1 to 10 equivalents and preferably 1 to 2 equivalents with respect to compound (V). The solvent is not particularly restricted so long as it does not affect the reaction, and it may be THF, DMF or 1,4-dioxane, and preferably 1,4-dioxane. The reaction temperature will usually be 0 to 25°C and is preferably 10 to 25°C. The reaction time will usually be 1 to 24 hours and is preferably 18 to 24 hours. So long as the reaction is not affected, N-methylpiperazine may be added to process the excess $Boc_2O$.

In the subsequent step E, compound (VI) is reacted with nickel sulfate hexahydrate, sodium peroxodisulfate and sodium hydroxide to produce compound (VII). In step E, the sodium peroxodisulfate is used at 1 to 4 equivalents and preferably 1 to 2 equivalents with respect to compound (VI). The nickel sulfate hexahydrate is used at 0.01 to 0.1 equivalent and preferably 0.08 to 0.1 equivalent with respect to compound (VI). The base used may be sodium hydroxide, at 1 to 10 equivalents and preferably 6 to 8 equivalents in total with respect to compound (VI). The solvent used is water. The reaction temperature is 10 to 40°C and preferably 10 to 25°C. The reaction time will usually be 1 to 24 hours and is preferably 12 to 24 hours.

In the subsequent step F, after reacting compound (VII) and TFA, the pH is adjusted to basicity and reaction is carried out with Fmoc-OSu to produce compound (VIII). The acid used in step F may be hydrochloric acid, sulfuric acid or trifluoroacetic acid, etc., and is preferably trifluoroacetic acid. The solvent is not particularly restricted so long as it does not affect the reaction, and it may be THF, dichloromethane or 1,4-dioxane, with dichloromethane being preferred. The reaction temperature is 0 to 25°C and preferably 10 to 25°C. The reaction time will usually be 0.5 to 5 hours and is preferably 0.5 to 2 hours. The inorganic base used to adjust the pH after the reaction, and as the base, may be sodium hydroxide, potassium hydroxide, sodium hydrogencarbonate, sodium carbonate or potassium carbonate, with potassium carbonate being preferred. Fmoc-OSu is used at 1 to 3 equivalents and preferably 1 to 1.5 equivalents with respect to compound (VII). The solvent is not particularly restricted so long as it does not affect the reaction, and it may be THF, N,N-dimethylformamide, dichloromethane or 1,4-dioxane, with 1,4-dioxane being preferred. The reaction temperature is 0 to 25°C and preferably 10 to 25°C. The reaction time will usually be 1 to 36 hours and is preferably 1 to 24 hours.

In the subsequent step G, compound (VIII) is reacted with trimethylsilyl azide and dibutyltin oxide in toluene to produce compound (IX). In step G, trimethylsilyl azide is used at 1 to 10 equivalents and preferably 2 to 4 equivalents with respect to compound (II). Dibutyltin oxide is used at 0.3 to 1 equivalent and preferably 0.7 to 1.0 equivalent with respect to compound (II). The solvent is not particularly restricted so long as it does not affect the reaction, and examples include benzene, toluene and xylene, with toluene being preferred. The reaction time will usually be 0.5 to 48 hours and is preferably 0.5 to 2 hours. The reaction time will usually be 80 to 150°C and is preferably 80 to 100°C. The heating method may be with an oil bath or microwave, with microwave heating being preferred.

In the subsequent step H, compound (IX) may be reacted with trityl chloride in the presence of a base to produce compound (X). The base used for production of compound (X) may be pyridine, 4-dimethylaminopyridine, triethylamine, N,N-diisopropylethylamine or N-methylmorpholine, with N-methylmorpholine being preferred. The amount of base used is 1 to 3 equivalents and preferably 1 to 1.5 equivalents with respect to compound (IX). It may be used at 1 to 3 equivalents and preferably 1.05 to 1.1 equivalents with respect to the trityl chloride compound (IX). The solvent is not particularly restricted so long as it does not affect the reaction, and examples include N,N-dimethylformamide, N,N-dimethyl acetamide, toluene, dichloromethane, tetrahydrofuran and acetonitrile, with tetrahydrofuran being preferred. The reaction temperature will usually be -10 to 60°C and is preferably 20 to 30°C, while the reaction time is 0.5 to 12 hours and preferably 1 to 2 hours.

Fmoc-(SOxa)-OH, having the carboxyl group of the aspartic acid replaced with 1,2,4-oxadiazol-5-one, can be produced by the method represented by Reaction Scheme 3 or a similar method.

[Chemical Formula 6]

Reaction Scheme 3

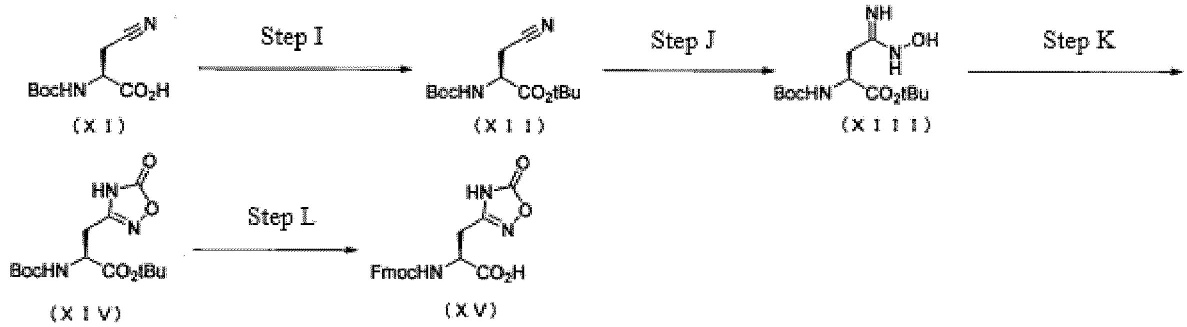


(XI) (XII) (XIII) (XIV) (XV)

In step I of the method of Reaction Scheme 3, a compound represented by the general formula of compound (XI) is reacted with 2-bromo-2-methylpropane in the presence of benzyltriethylammonium chloride and a base to produce compound (XII). In step I, the base is used at 10 to 30 equivalents and preferably 10 to 25 equivalents with respect to compound (XI). The base used may be potassium carbonate. Benzyltriethylammonium chloride is used at 1 to 3 equivalents and preferably 1 to 1.2 equivalents with respect to compound (XI). The 2-bromo-2-methylpropane is used at 10 to 50 equivalents and preferably 10 to 40 equivalents with respect to compound (XI). The solvent used is N,N-dimethyl acetamide. The reaction time will usually be 1 to 24 hours and is preferably 18 to 24 hours. The reaction temperature will usually be 25 to 70°C and is preferably 25 to 55°C.

In the subsequent step J, compound (XII) is reacted with hydroxylamine hydrochloride in the presence of a base to produce compound (XIII). The base used in step J may be triethylamine or N,N-diisopropylethylamine, and is preferably triethylamine. The base used with compound (XII) is used at 1 to 3 equivalents and preferably 1 to 2 equivalents. The hydroxylamine hydrochloride is used at 1 to 2 equivalents and preferably 1 to 1.5 equivalents with respect to compound (XII). The solvent is not particularly restricted so long as it does not affect the reaction, and examples include methanol, ethanol, propanol and isopropanol, with ethanol being preferred. The reaction temperature will usually be 80 to 100°C and is preferably 80 to 95°C. The reaction time will usually be 1 to 6 hours and is preferably 1 to 4 hours.

In the subsequent step K, compound (XIII) is reacted with carbonyldiimidazole to produce compound (XIV). In step K, the carbodiimidazole is used at 1 to 2 equivalents and preferably 1 to 1.2 equivalents with respect to compound (XIII). The solvent is not particularly restricted so long as it does not affect the reaction, and it may be THF or 1,4-dioxane, and preferably THF. The reaction temperature is 40 to 60°C and preferably 40 to 55°C. The reaction time will usually be 1 to 6 hours and is preferably 1 to 3 hours. For continuous cyclization reaction, the solvent is not particularly restricted so long as it does not affect the reaction, and it may be toluene, xylene or benzene, or preferably toluene. The reaction temperature will usually be 100 to 130°C and is preferably 100 to 120°C.

In the subsequent step L, the compound (XIV) is reacted with TFA in the presence of a scavenger, the pH is adjusted to basicity and reaction is conducted with Fmoc-OSu to produce compound (XV). The acid used in step L may be hydrochloric acid, sulfuric acid or trifluoroacetic acid, and is preferably trifluoroacetic acid. The scavenger used may be triethylsilane or triisopropylsilane, and is preferably triethylsilane. The solvent is not particularly restricted so long as it does not affect the reaction, and it may be THF, dichloromethane or 1,4-dioxane, with dichloromethane being preferred. The reaction temperature is 0 to 25°C and preferably 10 to 25°C. The reaction time will usually be 0.5 to 5 hours and is preferably 0.5 to 3 hours. The inorganic base used to adjust the pH after the reaction, and as the base, may be sodium hydroxide, potassium hydroxide, sodium hydrogencarbonate, sodium carbonate or potassium carbonate, with sodium hydrogencarbonate being preferred. Fmoc-OSu is used at 1 to 3 equivalents and preferably 1 to 1.5 equivalents with respect to compound (VII). The solvent is not particularly restricted so long as it does not affect the reaction, and it may be THF, N,N-dimethylformamide, dichloromethane or 1,4-dioxane, with 1,4-dioxane being preferred. The reaction temperature is 0 to 25°C and preferably 10 to 25°C. The reaction time will usually be 1 to 36 hours and is preferably 1 to 24 hours.

The peptide of the invention can exhibit physiological activity similar to PACAP, by binding with PAC1R, VPAC1R and/or VPAC2R. As specific examples, the peptide of the invention can exhibit physiological activity as a neuroprotective

substance, a nerve regeneration factor, a wound healing promoting factor, an inflammation suppressing factor or an exocrine secretagogue. According to another aspect, therefore, the invention may relate to a neuroprotective agent, a nerve-regenerating agent, a wound healing agent, a suppressing agent for inflammation, a corneal epithelial or corneal endothelial damage treatment agent, a dry-eye treatment agent, an exocrine gland secretagogue or a neurotrophic keratitis treatment agent, containing a peptide of the invention. According to yet another aspect, the invention relates to a pharmaceutical composition containing the peptide of the invention.

The neuroprotective agent of the invention is an agent having neuroprotective action. Therefore, a neuroprotective agent is one that can protect nerves from disorder brought on by neuronal damages, degeneration and/or cell death, and it can be a neuronal death (apoptosis and/or necrosis) inhibiting agent, a neuronal degeneration inhibiting agent, a neuronal stress reducing agent, a neurocytotoxicity resistance improving agent, a neuronal viability improving agent or an abnormal protein accumulation inhibiting agent.

Throughout the present specification, "neuroprotective action" refers to action of protecting neurons from their damage, degeneration and/or cell death, and preferably it is action of protecting them from neuronal death. More specifically, neuroprotective action may include inhibiting neuron death (apoptosis and/or necrosis), inhibiting neuronal degeneration, alleviating neuronal stress, increasing neurocytotoxicity resistance, improving neuron viability, or inhibiting accumulation of abnormal proteins. Neurons suffer damage due to physical damage, as well as exposure to neurotoxic substances and lack of oxygen or nutrients, and they can undergo cell death if a certain level of damage is exceeded. Neurons also suffer denaturation by accumulation of neurotoxic substances, eventually leading to cell death. Neurotoxic substances are largely classified as exogenous toxic substances or endogenous toxic substances. Exogenous toxic substances include heavy metals, alcohol, and chemical substances such as botulinum toxin. Endogenous toxic substances include active oxygen species, neurotransmitters such as glutamic acid, and abnormal proteins. Neuroprotective action can be easily measured by a person skilled in the art. As an example, neurons may be cultured in culture medium containing a test substance (drug group) or culture medium lacking the test substance (control group) under conditions with various types of stress, such as low oxygen load, exposure to neurotoxic substances, nutrient depletion or ultraviolet irradiation, measuring the viable cell count and dead cell count in each culture medium and calculating the percentage of viable cells with respect to the total number of cells, and the test substance can be assessed to have neuroprotective action if the viable cell percentage in the drug group is higher than the viable cell percentage in the control group. As a more preferred method, it can be measured by comparison with a positive control group containing an added substance known to have neuroprotective action, such as IGF-1 or NGF, determining whether or not the test substance has protective action equal to or greater than the positive control group. As another example, the neuroprotective action can be measured by an *in vivo* animal experiment.

The peptide of the invention has the exocrine gland secretagogue action of PACAP. The exocrine gland may be a lacrimal gland or salivary gland, and the peptide may be used as an exocrine gland secretagogue such as a lacrimal secretagogue promoting agent or salivary secretagogue. Without being limited to any particular theory, presumably PACAP and the peptide of the invention promote secretion of saliva or lacrimal fluid by binding to receptors (PAC1R, VPAC1R, VPAC2R) expressed on acinar cells of exocrine glands. Lacrimal fluid covers the surface of the keratoconjunctiva to maintain its wettability, with the lacrimal fluid filling the indentations formed by microvilli on the corneal surface and allowing clear images to be obtained. Epithelial cells of the keratoconjunctiva undergo active metabolism, and when unnecessary cells or metabolites are shed and discharged from the outermost surface, lacrimal fluid flushes them out while supplying the necessary oxygen and nutrients. Lacrimal fluid also flushes out contaminants that have infiltrated the keratoconjunctiva surface, while the bacteriostatic action of lacrimal fluid plays an important role for protection from infection by viruses, bacteria and fungi infiltrating from the outside environment. It also functions as synovial fluid between the eyelids and keratoconjunctiva, for smooth blinking and eye movement. Although the lacrimal fluid constitutes only a small amount of fluid forming a minute thin-film on the keratoconjunctiva surface, it is indispensable for maintaining corneal transparency and homeostasis by a variety of elaborate mechanisms. The conditions of an abnormal keratoconjunctiva surface due to disorder in secretion of lacrimal fluid is generally referred to as "dry-eye", and compounds have been created that promote lacrimal secretion when keratoconjunctival damage has occurred due to dry-eye, serving as prophylactic and therapeutic agents that are useful for dry-eye and conditions associated with dry-eye. The peptide of the invention can be included as a lacrimal secretagogue in an ophthalmic eye drop.

The peptide of the invention can also be used as a suppressing agent for inflammation, since it has the anti-inflammatory action of PACAP. With no intention to be limited to any particular theory, PACAP and the peptide of the invention are able to suppress production of inflammatory cytokines (TNF-$\alpha$, IL-6, IL-12, etc.). The peptide of the invention can therefore be used as an anti-inflammatory drug. The PACAP receptors VPAC1R and VPAC2R, in particular, are widely distributed in the gastrointestinal tract and also exhibit anti-inflammatory action, and can be used for treatment of inflammatory intestinal diseases, in particular.

According to another aspect, the invention relates to a pharmaceutical composition containing a therapeutically effective dose of the peptide described above. The pharmaceutical composition of the invention can be used for treatment or prevention of a disease that is improved by physiological action of PACAP. Diseases that are improved by physiological

action of PACAP include nervous disorder, diseases related to reduced lacrimal fluid, inflammatory diseases, and neurotrophic keratitis. The pharmaceutical composition of the invention can treat a disease that is improved by physiological action of PACAP, by administration to a patient, or it can prevent the disease by administration to a patient with the potential for developing the disease. Here, "treatment" means that after onset of a disorder or disease, worsening of the condition is prevented, or the current condition is maintained, alleviated or reversed, while "prevention" means that onset of a disorder or disease is prevented before onset.

Nervous disorder is a pathological condition where neuronal function is lost due to degeneration or cell death, and it includes cerebrovascular disorders and neurodegenerative disease.

Cerebrovascular disorders include hemorrhagic disorders, such as cerebral hemorrhage and subarachinoid hemorrhage, and cerebrovascular obstruction, such as cerebral thrombus, cerebral infarction and cerebral circulatory insufficiency. Both hemorrhagic disorders and obstructive disorders leave neurons in the brain in a state of hypoxia, leading to cell death. Therefore, the peptide, neuroprotective agent or pharmaceutical composition of the invention can be administered for the purpose of treatment or prevention of such cerebrovascular disorders.

Neurodegenerative diseases include, but are not limited to, degenerative diseases of the brain and central nervous system, such as dementia, Parkinson’s disease, spinocerebellar degeneration, Creutzfeldt-Jakob disease, Alzheimer’s disease, Huntington’s disease, multiple sclerosis, mad cow disease and epilepsy, motor neurodegenerative diseases such as spontaneous progressive muscular atrophy, amyotrophic lateral sclerosis and spinal-bulbar muscular atrophy, and sensory neurodegenerative diseases. Sensory neurodegenerative diseases include, but are not limited to, degenerative diseases of visual, auditory, tactile, gustatory and olfactory nerves, with visual degenerative diseases including glaucoma, retinitis pigmentosa, age-related macular degeneration and diabetic retinopathy, and auditory neurodegenerative diseases including hearing impairment.

Diseases related to reduced lacrimal fluid include, but are not limited to, dry-eye, keratoconjunctivitis sicca and reduced lacrimation.

Neurotrophic keratitis is a degenerative corneal disease resulting from damage to the trigeminal nerve, and they lead to damage of the corneal epithelium. The most severe type may lead to corneal ulceration, fusion or perforation, and loss of vision by the patient.

A vascular endothelial function ameliorator is a drug that improves vascular endothelial function. Vascular endothelial cells are cells on the innermost layer of blood vessels, which regulate adhesion of inflammatory cells on vascular walls, vascular permeability and the clotting/fibrinolytic system, which includes governance of vascular wall contraction and relaxation (hardness/softness of blood vessels). Because the peptide of the invention has a vasodilating effect as a physiological action of PACAP, it improves vascular endothelial disorders such as arteriosclerosis.

Corneal epithelial or endothelial disorders are conditions in which the corneal epithelial cells and corneal endothelial cells are impaired. Corneal epithelial cells are proliferative cells, but in cases of corneal epithelial cell impairment, their proliferation potency is suppressed, with progressive epithelial shedding leading to an imbalance in epithelial homeostasis. Corneal endothelial cells do not proliferate in the body and decrease with aging. Corneal epithelial or corneal endothelial damage means corneal epithelial or endothelial injury caused by intrinsic disease factors such as corneal ulcer, corneal epithelial detachment, diabetic keratopathy, keratoconjunctivitis sicca, chronic superficial keratitis, superficial punctate keratopathy, corneal erosion and protracted corneal epithelial loss, extrinsic diseases factors such as drugs, trauma or contact lens usage, or physical or chemical injury.

Dry-eye is a chronic disease of lacrimal fluid and the conjunctival epithelium due to a variety of factors, and it is accompanied by eye discomfort and visual function abnormalities. Lacrimal fluid abnormalities include the quantitative abnormality of reduced lacrimal fluid flow, and the qualitative abnormality of change in lacrimal fluid quality or ability to retain lacrimal fluid. Dry-eye also includes the dry-eye that accompanies reduced lacrimation, lacrimal fluid-evaporative dry eye, Sjogren’s syndrome, Stevens-Johnson syndrome, corneal epithelial erosion, blepharitis, ophthalmic pemphigus, vernal conjunctivitis, allergic conjunctivitis and vitamin A deficiency.

Inflammatory diseases include, but are not limited to, asthma, atopic dermatitis, hives, pollen hypersensitivity, anaphylactic shock, sinusitis (including eosinophilic sinusitis), rheumatism, multiple sclerosis, arthritis, systemic lupus erythematosus, psoriasis, ankylosing spondilitis, inflammatory intestinal disease (for example, ulcerative colitis, Crohn disease and gluten-sensitive intestinal disease), Sjogren’s syndrome, chronic graft-versus-host disease (GVHD), corneal infectious disease, allergic conjunctivitis, corneal trauma, polymyositis, dermatomyositis, myasthenia gravis, chronic obstructive pulmonary disease (COPD) and dermatosclerosis.

The peptide of the invention, or a neuroprotective agent, nerve-regenerating agent, suppressing agent for inflammation, wound healing promoter or exocrine gland secretagogue containing the peptide, or the pharmaceutical composition, may be administered parenterally or orally, depending on the disease to be treated. Oral administration may be sublingual, intraoral or oral administration. Examples of parenteral administration include administration by intravenous, intraarterial, subcutaneous, local, intraperitoneal, intramuscular, nasal, transdermal, transmucosal, intrameningeal, perrectal, intramuscular, intracerebral, intrameningeal, subarachnoid, intradural, epidural, eye drop, ear drop, nasal drop or intraocular routes. Intraocular routes include, more specifically, subconjunctival, sub-tenon and intravitreal

routes. A pharmaceutical composition containing the peptide of the invention may be prepared in an appropriate dosage form for the route of administration, and may be an eye drop, injection, powdered drug, infusion preparation, granules, tablets or suppository, for example, but for parenteral administration it is preferably an eye drop, injection or infusion preparation, or a powdered drug for preparation prior to use. A formulation for intraocular administration may be an intravitreal injection, subconjunctival injection or sub-tenon injection, for example. Such formulations may also contain various pharmaceutically acceptable adjuvants, i.e. additives such as carriers or other auxiliary agents, which include stabilizers, antiseptic agents, soothing agents and emulsifiers. They may also be used in combination with other drugs that have neuroprotective effects, inflammation-suppressing effects or exocrine gland secretion effects.

The examples of the invention described below are intended to serve merely as illustration and do not limit the technical scope of the invention. The technical scope of the invention is limited solely by the description in the Claims.

EXAMPLES

Example 1A: Synthesis of Fmoc-(D)-Tz(Trt)-OH (compound IV)

Fmoc-D-Tz(Trt)-OH (compound IV) was synthesized according to the following reaction scheme.

[Chemical Formula 7]

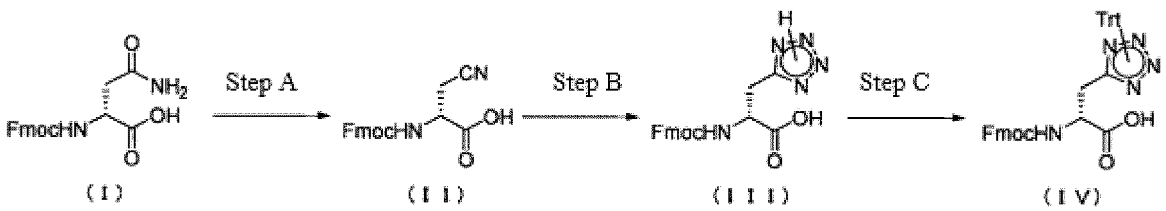


Step A: Synthesis of Fmoc-D-Ala(β-CN)-OH:

[Chemical Formula 8]

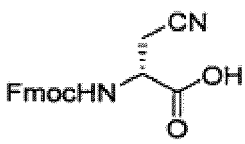

Compound (II): Fmoc-D-Ala(β-CN)-OH

After adding a solution of pyridine (30 mL) and 1-(3-dimethylaminopropyl)-3-ethylcarbodiimide hydrochloride (3.96 g, 21 mmol) suspended in acetonitrile (35 mL) to a solution of Fmoc-D-Asn-OH (5.04 g, 14 mmol) in acetonitrile (35 mL), at 0°C, the mixture was stirred for 4 hours at room temperature. The reaction mixture was concentrated under reduced pressure, ethyl acetate was added to the residue, the mixture was washed with 3 M hydrochloric acid and brine and dried over sodium sulfate, and the solvent was removed by distillation under reduced pressure. The obtained crude product was dissolved in acetone and hexane/diethyl ether (1/1) was added to produce a precipitate, which was then concentrated under reduced pressure and filtered to obtain the target compound (4.76 g, 99%) as a white solid.
$^{1}H$ NMR (400 MHz, DMSO-$d_6$) δ 13.20 (1H, brs), 8.08 (1H, d, J = 8.4 Hz), 7.90 (2H, d, J = 7.6 Hz), 7.72 (2H, d, J = 7.2 Hz), 7.42 (2H, t, J = 7.2 Hz), 7.33 (2H, t, J = 7.6 Hz), 4.39-4.32 (3H, m), 4.25 (1H, t, J = 7.2 Hz), 2.98 (1H, dd, J = 16.8, 4.8 Hz), 2.88 (1H, dd, J = 16.8, 9.2 Hz).

Step B: Synthesis of Fmoc-D-Tz-OH:

[Chemical Formula 9]

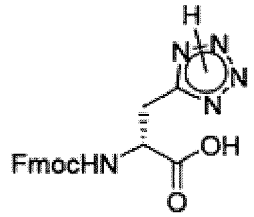

Compound (III): Fmoc-D-Tz-OH:

In the structural formula shown above, H is bonded to either N1 or N2 on the tetrazole ring.

After adding dibutyltin oxide (0.710 g, 2.9 mmol) and trimethylsilyl azide (1.8 mL, 14 mmol) to a solution of Fmoc-D-Ala(β-CN)-OH (1.01 g, 3.0 mmol) in toluene (18 mL), the mixture was heated in a microwave at 100°C for 1.5 hours. The reaction mixture was returned to room temperature and filtered. The obtained filtered substance was washed with toluene and hexane and dissolved in ethanol/dichloromethane (3/2.25 mL), and then cooled to 4°C, after which 1 M hydrochloric acid (5 mL) was added and the mixture was stirred for 2 hours and 15 minutes at room temperature. The reaction mixture was washed with 1 M hydrochloric acid and brine, the solvent was removed by distillation under reduced pressure, and then hexane was added to form a suspension which was filtered to obtain a pale yellow solid of the target compound (1.03 g, 90%).

$^1$H NMR (400 MHz, DMSO-$d_6$) δ 16.00 (1H, brs), 13.07 (1H, brs), 7.89 (2H, d, H = 7.6 Hz), 7.83 (1H, d, J = 8.4 Hz), 7.65 (2H, t, J = 8.0 Hz), 7.41 (2H, t, J = 7.6 Hz), 7.31 (2H, t, J = 7.6 Hz), 4.53-4.48 (1H, m), 4.27-4.18 (3H, m), 3.40 (1H, dd, J = 15.2, 6.0 Hz), 3.28 (1H, dd, J = 15.2, 8.8 Hz).

Step C: Synthesis of Fmoc-D-Tz(Trt)-OH:

[Chemical Formula 10]

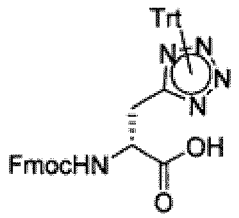

Compound (IV): Fmoc-D-Tz(Trt)-OH

In the structural formula shown above, a Trt group is bonded to N1 or N2 on the tetrazole ring.

After adding N-methylmorpholine (0.72 mL, 7.2 mmol) and trityl chloride (1.63 g, 5.9 mmol) to a solution of Fmoc-D-Tz-OH (1.99 g, 5.3 mmol) in THF (10 mL), the mixture was stirred for 1.5 hours at room temperature. The reaction mixture was filtered and the filtered substance was washed with THF and combined with the filtrate, after which the solvent was removed by distillation under reduced pressure. The residue was purified by column chromatography (ODS, $H_2O/CH_3CN$ = 80/20 -> 30/70 -> 20/80), the fraction containing the target compound was extracted with ethyl acetate, washed with brine and dried over sodium sulfate, and then the solvent was removed by distillation under reduced pressure and vacuum drying was carried out at 60°C to obtain the target compound (1.29 g, 40%) as a white solid. $^1$H NMR (400 MHz, DMSO-$d_6$) δ 13.03 (1H, brs), 7.91 (2H, d, J = 7.6 Hz), 7.79 (1H, d, J = 8.8 Hz), 7.67 (2H, brd, J = 6.8 Hz), 7.44-7.23 (13H, m), 7.01-6.99 (6H, m), 4.52-4.46 (1H, m), 4.28-4.15 (3H, m), 3.40 (1H, dd, J = 15.2,5.2 Hz), 3.27 (1H, dd, J = 15.2,9.2 Hz).

Example 1B: synthesis of Fmoc-nvTz(Trt)-OH (compound X)

Fmoc-nvTz(Trt)-OH (compound X) was synthesized according to the following reaction scheme.

[Chemical Formula 11]

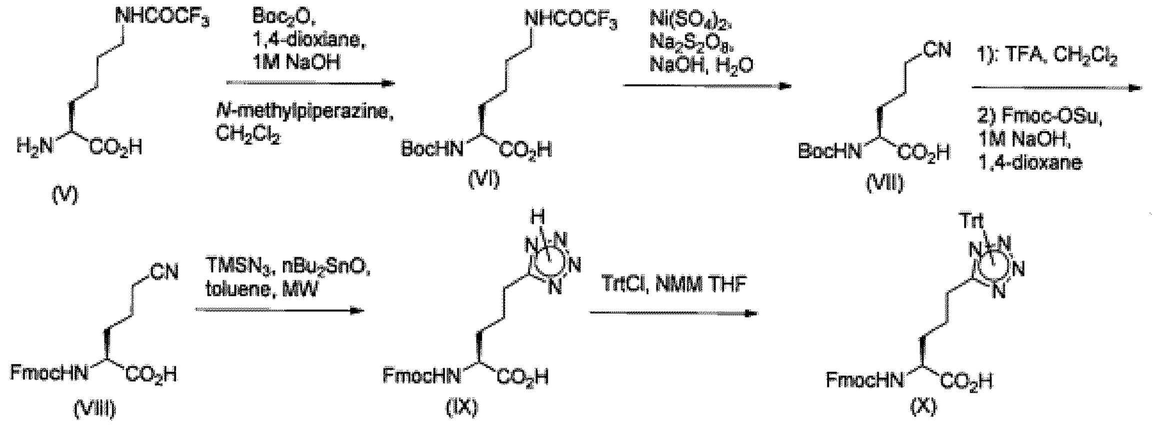


Step D: Synthesis of Boc-Nε-trifluoroacetyl-L-lysine:

[Chemical Formula 12]

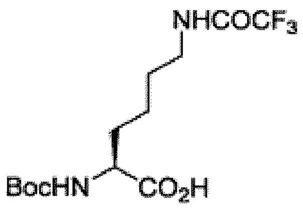

Compound (VI): Boc-$N^{\varepsilon}$-trifluoroacetyl-L-lysine

After adding a 1 M sodium hydroxide aqueous solution (42 mL, 42 mmol) and $Boc_2O$ (30% THF solution, 49.8 g, 68 mmol) to a solution of Nε-trifluoroacetyl-L-lysine (10.0 g, 41 mmol) in 1,4-dioxane (70 mL), the mixture was stirred for 1 day at room temperature. After then adding N-methylpiperazine (3.5 mL) to the reaction mixture and stirring for 2 hours at room temperature, 1 M hydrochloric acid was added to adjust the reaction mixture to pH 2, and it was extracted with ethyl acetate and washed with brine. The organic layer was dried over sodium sulfate and the solvent was removed by distillation under reduced pressure to obtain the title compound (15.9 g, >100%) as a white solid.
$^1$H NMR (400 MHz, $CDCl_3$) δ 6.69 (1H, brs), 5.12 (1H, d, J = 6.8 Hz), 4.30 (1H, brs), 3.38 (2H, q, J = 6.8 Hz), 1.94-1.86 (1H, m), 1.74-1.57 (3H, m), 1.52-1.41 (3H, m), 1.45 (9H, s).

Step E: Synthesis of (S)-2-[(*tert*-butoxycarbonyl)amino]-5-cyanopentanoic acid

[Chemical Formula 13]

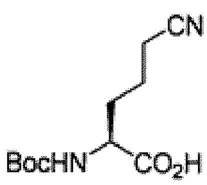

Compound (VII): (S)-2-[(*tert*-Butoxycarbonyl)amino]-5-cyanopentanoic acid

After adding Boc-Ne-trifluoroacetyl-L-lysine (1.00 g, 2.9 mmol), sodium hydroxide (276.4 mg, 6.9 mmol) and

water (15 mL) to a flask, a nickel(II) sulfate hexahydrate (77.0 mg, 0.29 mmol) aqueous solution (1.0 mL), sodium peroxodisulfate (1.43 g, 6.0 mmol) and sodium hydroxide (529.5 mg, 13 mmol) were added and the mixture was stirred for 19.5 hours at room temperature. The reaction mixture was adjusted to pH 2 with 1 M hydrochloric acid and extracted with ethyl acetate, the obtained organic layer was washed with brine and dried over sodium sulfate, and the solvent was removed by distillation under reduced pressure. The residue was purified by silica gel column chromatography (dichloromethane/methanol = 100/0 -> 95/5) to obtain the title compound (661.5 mg, 86%) as a white solid.
$^1H$ NMR (400 Mz, DMSO-$d_6$) δ 12.53 (1H, brs), 7.14 (1H, d, J = 8.4 Hz), 3.93-3.88 (1H, m), 2.53-2.48 (2H, m), 1.81-1.74 (1H, m), 1.69-1.56 (3H, m), 1.39 (9H, m)

Step G: Synthesis of (S)-2-({[(9H-fluoren-9-yl)methoxy]carbonyl]amino)-5-cyanopentanoic acid:

[Chemical Formula 14]

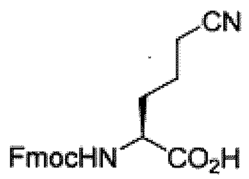

Compound (VIII): (S)-2-({[(9H-Fluoren-9-yl)methoxy]carbonyl}amino)-5-cyanopentanoic acid

A solution of (S)-2-[(tert-butoxycarbonyl)amino]-5-cyanopentanoic acid (586.3 g, 2.4 mmol) in dichloromethane (2.5 mL) was cooled to 0°C, and then trifluoroacetic acid (2.5 mL) was added and the mixture was stirred for 1 hour at room temperature. The solvent of the reaction mixture was removed by distillation under reduced pressure. After adding 1,4-dioxane (3.0 mL) to the obtained residue, a 10% potassium carbonate aqueous solution and solid potassium carbonate were added to pH 9, and then Fmoc-OSu (1.26 g, 3.7 mmol) was added and the mixture was stirred for 2 hours at room temperature. The reaction mixture was diluted with an ethyl acetate/hexane (1/1) solution and washed with 0.5 M hydrochloric acid and brine, and then the obtained organic layer was dried over sodium sulfate and the solvent was removed by distillation under reduced pressure. The obtained residue was purified by silica gel column chromatography (ethyl acetate/hexane = 10/90 -> 31/69) to obtain the title compound (731.1 mg, 83%) as a white solid.
$^1H$ NMR (4000 MHz, $CDCl_3$) δ 7.76 (2H, d, J = 7.2 Hz), 7.58 (2H, brd, J = 6.8 Hz), 7.40 (2H, t, J = 7.2 Hz), 7.31 (2H, t, J = 6.8 Hz), 5.38 (1H, d, J = 7.6 Hz), 4.64-4.36 (3H, m), 4.20 (1H, J = 6.4 Hz), 2.38 (1H, brt, J = 6.4 Hz), 2.28-2.07 (2H, m), 1.88-1.41 (3H, m)

Step H: Synthesis of Fmoc-nvTz-OH

[Chemical Formula 15]

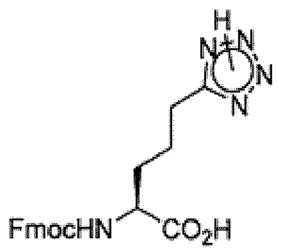

Compound (IX): Fmoc-nvTz-OH

In the structural formula shown above, H is bonded to either N1 or N2 on the tetrazole ring.
After adding trimethylsilyl azide (1.2 mL, 9.2 mmol) to a solution of (S)-2-({[(9H-fluoren-9-yl)methoxy]carbonyl}amino)-5-cyanopentanoic acid (851 mg, 2.3 mmol) and dibutyltin oxide (581 mg, 2.3 mmol) in toluene (17 mL), the mixture was heated in a microwave at 100°C for 1.5 hours. The reaction mixture was returned to room temperature and

filtered, and the obtained solid was washed with toluene and hexane to obtain a pale orange solid. After adding ethanol/dichloromethane (3/2.50 mL) to the solid and cooling to 0°C, 1 M hydrochloric acid (5.0 mL) was added and the mixture was stirred for 2 hours at room temperature. Ethyl acetate was added to the reaction mixture and the resulting mixture was washed with 1 M hydrochloric acid and brine and dried over sodium sulfate, and the solvent was removed by distillation under reduced pressure. The obtained residue was purified by silica gel column chromatography (dichloromethane/methanol = 100/0 -> 95/5) to obtain the title compound (367 mg, 39%). $^1$H NMR (400 MHz, DMSO-$d_6$) δ 7.88 (2H, d, J = 7.6 Hz), 7.72 (2H, d, J = 7.2 Hz), 7.63 (1H, d, J = 7.6 Hz), 7.40 (2H, t, J = 7.6 Hz), 7.31 (2H, t, J = 7.2 Hz), 4.29-4.20 (3H, m), 4.01-3.96 (1H, m), 2.89 (2H, m), 1.78-1.46 (5H, m).

Step I: Synthesis of Fmoc-nvTz(Trt)-OH

[Chemical Formula 16]

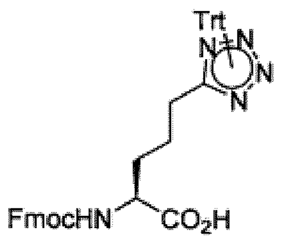

Compound (X): Fmoc-nvTz(Trt)-OH

In the structural formula shown above, a Trt group is bonded to either N1 or N2 on the tetrazole ring.

After adding N-methylmorpholine (110 μL, 1.0 mmol) and trityl chloride (261 mg, 0.94 mmol) to a solution of Fmoc-nvTz-OH (365 mg, 0.89 mmol) in THF (4.0 mL), the mixture was stirred for 2 hours at room temperature. Upon completion of the reaction and filtering, the solvent of the obtained filtrate was distilled off under reduced pressure to obtain the title compound (540 mg, 93%) as a white solid.
$^1$H NMR (400 MHz, DMSO-$d_6$) δ 12.67 (1H, s), 7.89 (2H, d, J = 7.2 Hz), 7.72 (2H, m), 7.66 (1H, brs), 7.39-7.19 (13H, m), 7.02-6.98 (6H, m), 4.34-4.20 (3H, m), 3.98-3.94 (1H, m), 2.88-2.83 (2H, m), 1.77-1.62 (4H, m)

Example 1C: Synthesis of (S)-2-({[(9H-fluoren-9-yl)methoxy]carbonyl}amino)-3-(5-oxo-4,5-dihydro-1,2,4-oxadiazol-3-yl)propanoic acid (compound XV)

The compound (S)-2-({[(9H-fluoren-9-yl)methoxy]carbonyl}amino)-3-(5-oxo-4,5-dihydro-1,2,4-oxadiazol-3-yl)propanoic acid (compound XV) was synthesized according to the following reaction scheme.

[Chemical Formula 17]

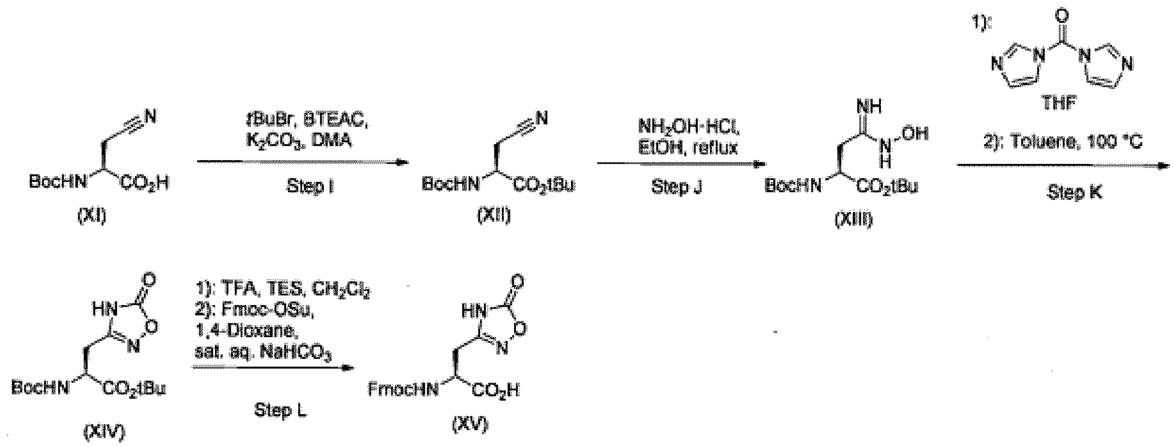


Step I: Synthesis of *tert*-butyl (S)-2-[(*tert*-butoxycarbonyl)amino]-3-cyanopropanoate

[Chemical Formula 18]

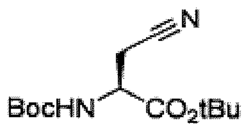

Compound (XII): *tert*-Butyl (S)-2-[(*tert*-butoxycarbonyl)amino]-3-cyanopropanoate

After adding 2-bromo-2-methylpropane (46 ml, 409 mmoL) to a suspension of Boc-Ala(CN)-OH (2.23 g, 10.4 mmol), benzyltriethylammonium chloride (2.38 g, 10.4 mmol) and potassium carbonate (37.0 g, 268 mmol) in DMA (60 mL), the mixture was stirred for 1 day at 55°C. The reaction mixture was returned to room temperature, water was added, extraction was performed with ethyl acetate and the obtained organic layer was washed with water and brine and dried over sodium sulfate, and then the solvent was removed by distillation under reduced pressure. The obtained residue was purified by flash silica gel column chromatography (hexane/ethyl acetate = 1/0 -> 1/4) to obtain the title compound (2.79 g, 99%) as a colorless oil. $^1$H NMR (400 MHz, $CDCl_3$) δ 5.45 (1H, brd, J = 4.8 Hz), 4.37 (1H, brq, J = 5.2 Hz), 2.94 (1H, dd, J = 16.8, 5.2 Hz), 2.88 (1H, dd, J = 16.8, 4.8 Hz), 1.51 (9H, s), 1.45 (9H, s).

Step J: Synthesis of *tert*-butyl (S)-2-[(*tert*-butoxycarbonyl)amino-4-(hydroxyamino)-4-iminobutanoate

[Chemical Formula 19]

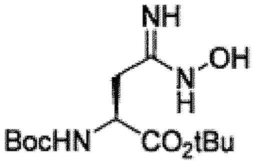

Compound (XIII): *tert*-Butyl (S)-2-[(*tert*-butoxycarbonyl)amino-4-(hydroxyamino)-4-iminobutanoate

After adding triethylamine (2.8 mL, 20 mmol) and hydroxylamine hydrochloride (1.08 g, 16 mmol) to a solution of *tert*-butyl (S)-2-[(*tert*-butoxycarbonyl)amino]-3-cyanopropanoate (2.79 g, 10 mmol) in ethanol (30 mL), the mixture was stirred for 4 hours at 95°C. The reaction mixture was returned to room temperature, water was added, the mixture

was extracted with ethyl acetate and dried over sodium sulfate, and the solvent was removed by distillation under reduced pressure and vacuum dried to obtain the title compound (2.85 g, 91%) as a white amorphous solid.
$^1$H NMR (400 MHz, $CDCl_3$) δ 6.47 (1H, brs), 5.47 (1H, brs), 4.63 (2H, brs), 4.37 (1H, brs), 2.62-2.54 (2H, m), 1.46 (9H, s), 1.44 (9H, s).

Step K: Synthesis of *tert*-butyl (S)-2-[(*tert*-butoxycarbonyl)amino]-3-(5-oxo-4,5-dihydro-1,2,4-oxadiazol-3-yl)propanoate

[Chemical Formula 20]

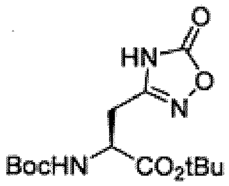

Compound (XIV): *tert*-Butyl (S)-2-[(*tert*-butoxycarbonyl)amino]-3-(5-oxo-4,5-dihydro-1,2,4-oxadiazol-3-yl)propanoate

After adding 1,1-carbonyldiimidazole (627 mg, 3.9 mmol) to a solution of tert-butyl (S)-2-[(*tert*-butoxycarbonyl)amino]-4-(hydroxyamino)-4-iminobutanoate (1.04 g, 3.4 mmol) in THF (20 mL), the mixture was stirred for 1.5 hours at room temperature. The reaction mixture was concentrated, toluene (20 mL) was added and the mixture was stirred for 2 hours at 100°C. The reaction mixture was returned to room temperature, the solvent was removed by distillation under reduced pressure, and the obtained residue was purified by flash silica gel column chromatography (hexane/ethyl acetate = 1/1) to obtain the title compound (658.9 mg, 58%) as a white amorphous solid.
$^1$H NMR (400 MHz, DMSO-d $_6$) δ 12.23 (1H, brs), 7.36 (1H, d, J = 8.0 Hz), 4.24 (1H, q, J = 8.0 Hz), 2.90 (1H, dd, J = 15.2, 7.2 Hz), 2.81 (1H, dd, J = 15.2, 8.4 Hz), 1.38 (9H, s), 1.37 (9H, s).

Step L: Synthesis of (S)-2-({[(9H-fluoren-9-yl)methoxy]carbonyl}amino)-3-(5-oxo-4,5-dihydro-1,2,4-oxadiazol-3-yl)propanoic acid

[Chemical Formula 21]

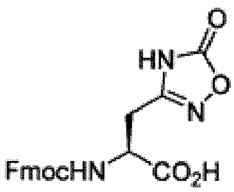

Compound (XV): (S)-2-({[(9H-Fluoren-9-yl)methoxy]carbonyl}amino)-3-(5-oxo-4,5-dihydro-1,2,4-oxadiazol-3-yl)propanoic acid

After adding triethylsilane (1.0 mL) and trifluoroacetic acid (5.0 mL) to a solution of *tert*-butyl(S)-2-[(*tert*-butoxycarbonyl)amino]-3-(5-oxo-4,5-dihydro-1,2,4-oxadiazol-3-yl)propanoate (658.9 mg, 2.0 mmol) in dichloromethane (4.0 mL) at 0°C, the mixture was stirred for 3 hours at room temperature. The solvent was removed by distillation under reduced pressure, diethyl ether and hexane were added, the solvent was again removed by distillation under reduced pressure, 1,4-dioxane (10 mL) was added, and a saturated sodium hydrogencarbonate aqueous solution was added to pH 9. Next, Fmoc-OSu (813 mg, 2.4 mmol) was added at 0°C and the mixture was stirred overnight at room temperature. After adding a 10% citric acid aqueous solution to the reaction mixture for adjustment to pH 3, it was extracted with ethyl acetate and dried over sodium sulfate, the solvent was removed by distillation under reduced pressure, and the obtained residue was purified by silica gel column chromatography (dichloromethane/methanol = 100/0 -> 93/7) to obtain the title compound (465.3 mg, 59%) as a white amorphous solid.

$^1H$ NMR (400 MHz, DMSO-$d_6$) δ 7.89 (2H, d, J = 7.2 Hz), 7.70-7.63 (3H, m), 7.42 (2H, t, J = 7.2 Hz), 7.32 (2H, t, J = 7.2 Hz), 4.33-4.20 (4H, m), 2.96 (1H, dd, J = 15.2, 5.6 Hz), 2.80 (1H, dd, 15.2, 8.8 Hz),

Example 2: Peptide synthesis

The peptide used in the test was synthesized using a peptide synthesizer (model: PSSM-8 by Shimadzu Corp.), using solid phase synthesis by the Fmoc method. The non-natural amino acids Fmoc-Tz-OH, Fmoc-Tz(trt)-OH and Fmoc-egTz(trt)-OH used for solid phase synthesis were purchased from Astatech Co., Fmoc-cya-OH was purchased from Anaspec Co., and compounds (IV), (X) and (XV) synthesized in Example 1 were used. F-moc-Ai-OH and Fmoc-Cn-OH were purchased from Watanabe Chemical Engineering, and peptides 1 to 28 having the following sequences were synthesized. The molecular weights of the synthetic peptides were analyzed by mass spectrometry (MALDI TOF). As shown in Table 2, all of the measured values matched theoretical values. N-terminal and C-terminal modification were carried out by methods known to those skilled in the art.

[Table 2-1]

Table 2

| | N-terminus | Sequence | C-terminus | Theoretical value $[M+H]^+$ | Measured Value |
|---|---|---|---|---|---|
| Peptide 1 | H | HSTzGI FATzAY SRYRK QMAVK KYLA A VL (Seq ID No.4) | NH2 | 3148.70 | 3148.2 |
| Peptide 2 | H | HSTzGI FATzSY ARYRK QMAVK KYLA A VL (Seq ID No.5) | NH2 | 3148.70 | 3148.6 |
| Peptide 3 | H | HSTzGI FATzAY ARYRK QMAVK KYLA A VL (Seq ID No.6) | NH2 | 3132.70 | 3132.6 |
| Peptide 4 | H | HSTzGI FATzAiY SRYRK QMAVK KYL AA VL (Seq ID No.7) | NH2 | 3162.71 | 3162.3 |
| Peptide 5 | H | HSTzGI FATzSY AiRYRK QMAVK KYL AA VL (Seq ID No.8) | NH2 | 3162.71 | 3162.9 |
| Peptide 6 | H | HSTzGI FATzAiY AiRYRK QMAVK KY LAA VL (Seq ID No.9) | NH2 | 3160.73 | 3161.2 |
| Peptide 7 | H | HSTzGI FTTzAiY AiRYRK QMAVK KY LAA VL (Seq ID No.10) | NH2 | 3190.74 | 3190.3 |
| Peptide 8 | H | HSTzGI FTTzSY SRYRK QMAVK KYLA (Seq ID No.11) | NH2 | 2911.51 | 2911.2 |
| Peptide 9 | H | HSTzGI FTTzSY SRYRK QMAVK KYLA A V (Seq ID No.12) | NH2 | 3081.62 | 3081.4 |
| Peptide 10 | H | HScyaGI FTcyaSY SRYRK QLAVK KY LAA VL (Seq ID No.13) | NH2 | 3200.63 | 3200.9 |

(continued)

Table 2

| | N-terminus | Sequence | C-terminus | Theoretical value [M+H] + | Measured Value |
|---|---|---|---|---|---|
| Peptide 11 | H | HScyaGI FTcyaSY SRYRK QNIAVK K YLAA VL (Seq ID No.14) | NH2 | 3200.63 | 3201.0 |
| Peptide 12 | H | MHSTzGI FTTzSY SRYRK QLAVK KYL AA VL (Seq ID No.15) | NH2 | 3307.79 | 3307.3 |
| Peptide 13 | H | HSTz(D)GI FTTz(D)SY SRYRK QNIA VK KYLAA VL (Seq ID No.16) | NH2 | 3176.75 | 3176.8 |
| Peptide 14 | H | HSTzGI FTTz(D)SY SRYRK QNIAVK KYLAA VL (Seq ID No.17) | NH2 | 3176.75 | 3176.6 |
| Peptide 15 | H | HSTz(D)GI FTTzSY SRYRK QNIAVK KYLAA VL (Seq ID No.18) | NH2 | 3176.75 | 3176.8 |
| Peptide 16 | H | HSTzGI FTegTzSY SRYRK QNIAVK K YLAA VL (Seq ID No.19) | NH2 | 3190.76 | 3191.1 |
| Peptide 17 | $CH_3CH_2CO$ | HSTzGI FTTzSY SRYRK QNIAVK KYL AA VL (Seq ID No.20) | NH2 | 3232.77 | 3232.5 |
| Peptide 18 | $CH_3CH_2CH_2CO$ | HSTzGI FTTzSY SRYRK QNIAVK KYL AA VL (Seq ID No.21) | NH2 | 3246.79 | 3246.3 |
| Peptide 19 | $(CH_3)_2CH$ CO | HSTzGI FTTzSY SRYRK QNIAVK KYL AA VL (Seq ID No.22) | NH2 | 3246.79 | 3246.3 |
| Peptide 20 | $CH_3CH_2CH_2CH_2CO$ | HSTzGI FTTzSY SRYRK QNIAVK KYL AA VL (Seq ID No.23) | NH2 | 3260.80 | 3260.8 |

[Table 2-2]

| | N-terminus | Sequence | C-terminus | Theoretical value [M+H] + | Measured Value |
|---|---|---|---|---|---|
| Peptide 21 | $(CH_3)_2CH CH_2CO$ | HSTzGI FTTzSY SRYRK QNIAVK KYL AA VL (Seq ID No.24) | NH2 | 3260.80 | 3260.5 |
| Peptide 22 | Ac ($CH_3C$ 0) | HS(50xa)GI FT(50xa)SY SRYRK QN IAVK KYLAA VL (Seq ID No.25) | NH2 | 3250.72 | 3250.3 |
| Peptide 23 | Ac | HSTzGA FTTzSY SRYRK QLAVK KYLA A VL (Seq ID No.26) | NH2 | 3176.71 | 3176. 2 |
| Peptide 24 | Ac | HSTzGI FATzSY SRYRK QLAVK KYLA A VL (Seq ID No.27) | NH2 | 3188.75 | 3188.8 |
| Peptide 25 | Ac | HATzGI FATzSY SRYRK QLAVK KYLA A VL (Seq ID No.28) | NH2 | 3172.75 | 3172.4 |
| Peptide 26 | Ac | HATzGI FTTzSY SRYRK ALAVK KYLA A VL (Seq ID No.29) | NH2 | 3146.74 | 3146.2 |
| Peptide 27 | Ac | HSTzGI FATzAY SRYRK QLAVK KYLA A VL (Seq ID No.30) | NH2 | 3172.75 | 3172.4 |
| Peptide 28 | Ac | HSTzGI FATzSY SRYRK ALAVK KYLA A VL (Seq ID No.31) | NH2 | 3131.72 | 3131.2 |
| Peptide 29 | Ac | HSTzGI FATzSY SRYRK QAAVK KYLA A VL (Seq ID No.32) | NH2 | 3146.70 | 3147.0 |
| Peptide 30 | Ac | HATzGI FATzAY SRYRK AAAVK KYLA A VL (Seq ID No.33) | NH2 | 3057.69 | 3057.5 |
| Synthetic Example 1 | H | HSTzGI FATzSY CnRYRK QMAVK KYL AA VL (Seq ID No.34) | NH2 | 3173.69 | 3173.3 |

(continued)

| | N-terminus | Sequence | C-terminus | Theoretical value [M+H] + | Measured Value |
|---|---|---|---|---|---|
| Synthetic Example 2 | H | HSTzGI FTTzCnY CnRYRK QMAVK KY LAA VL (Seq ID No.３５) | NH2 | 3212.70 | 3212.7 |
| Synthetic Example 3 | H | HSegTzGI FTTzSY SRYRK QNIAVK K YLAA VL (Seq ID No.３６) | NH2 | 3190.76 | 3190.9 |
| Synthetic Example 4 | H | HSegTzGI FTegTzSY SRYRK QNIAVK KYLAA VL (Seq ID No.３７) | NH2 | 3204.78 | 3204.8 |
| Synthetic Example 5 | H | HSnvTzGI FTTzSY SRYRK QNIAVK K YLAA VL (Seq ID No.３８) | NH2 | 3204.78 | 3204.9 |
| Synthetic Example 6 | H | HSTzGI FTnvTzSY SRYRK QNIAVK K YLAA VL (Seq ID No.３９) | NH2 | 3204.78 | 3204.8 |
| Synthetic Example 7 | H | HSnvTzGI FTnvTzSY SRYRK QNIAVK KYLAA VL (Seq ID No.４０) | NH2 | 3232.81 | 3232.8 |
| Synthetic Example 8 | H | HSegTzGI FTnvTzSY SRYRK QNIAVK KYLAA VL (Seq ID No.４１) | NH2 | 3218.79 | 3218.4 |
| Synthetic Example 9 | H | HSnvTzGI FTegTzSY SRYRK QNIAVK KYLAA VL (Seq ID No.４２) | NH2 | 3218.79 | 3218.7 |

[Table 2-3]

| | N-terminus | Sequence | C-terminus | Theoretical value[M+H]+ | Measured Value |
|---|---|---|---|---|---|
| Reference Example 1 (PACAP27) | H | HSDGIFTDSYSRYRKQMAVKKYLAAVL (Seq ID No. 2) | NH2 | 13146.66 | 3147.0 |
| Reference Example 2 | Ac | HSDGIFTDSYSRYRKQMAVKKYLAAVL (Seq ID No. 51) | NH2 | 3188.67 | 3188.4 |
| Reference Example 3 | H | HSTzGIFTTzSYSRYRKQNIAVKKYLAAVL (Seq ID No. 52) | NH2 | 3176.75 | 3177.0 |
| Reference Example 4 | H | HSTzGIFTTzSYSRYRKQLAVKKYLAAVL (Seq ID No. 53) | NH2 | 3176.75 | 3217.9 |
| Reference Example 5 | Ac | HSTzGIFTTzSYSRYRKQNIAVKKYLAAVL (Seq ID No. 54) | NH2 | 3218.76 | 3219.2 |
| Reference Example 6 | Ac | HSTzGIFTTzSYSRYRKQLAVKKYLAAVL (Seq ID No. 55) | NH2 | 3218.76 | 3218.2 |

In the table, Tz represents tetrazole-substituted aspartic acid, egTz represents tetrazole-substituted glutamic acid, nvTz represents tetrazole-substituted homoglutamic acid, cya represents sulfoxy-substituted aspartic acid, (5Oxa) represents oxadiazolone-substituted aspartic acid, Ai represents α-aminoisobutyric acid and Cn represents β-cyanoalanine. Tz(D) indicates that tetrazole-substituted aspartic acid is the D-form.

The Tz introduced for peptides 1 to 9 was the racemic form. The amino acids used for the other peptides were L-forms unless otherwise specified.

Example 3: Stability test 1

[Measuring sample preparation 1]

The peptides synthesized in Example 2 (peptides 1 to 9) were weighed and dissolved in 0.1% phosphate buffer (pH 7.0) to prepare 1.0 mM peptide solutions. The 1.0 mM peptide solutions were then diluted with phosphate buffer to 100 μM. After filtering with Chromatdisk (product of Merck Millipore, Millex-GV, 0.22 μm), each filtrate was dispensed into an LC vial (Waters Deactivated Qsert Vial). The prepared peptide solutions were incubated for one month or 2 months in a 40°C thermostatic bath to obtain stored samples. A simultaneously prepared sample among the peptide solutions that was not stored was used as a standard sample (initial sample). The standard sample and the stored samples were stored at -30°C until sample analysis.

[Moisture permeability]

Using the total weight of the aqueous peptide solution and storage vessel as the specimen weight, measurements were recorded as the specimen weights before storage and the specimen weights after storage under different storage conditions. The empty weight of the storage vessel was also measured, and the moisture permeability was determined by the following formula.

[Mathematical Formula 1]

$$\text{Vessel moisture permeability (\%)} = \frac{\text{Specimen weight before storage (g)} - \text{specimen weight after storage (g)}}{\text{Specimen weight before storage (g)} - \text{storage vessel weight (g)}} \times 100$$

After stirring the standard sample and the stored samples with a vortex mixer, each peptide solution was transferred to an HPLC vial (Deactivated Qsert vial by Waters Co.). Reverse-phase HPLC (HPLC system: Prominence by Shimadzu Corp.) was conducted under the conditions shown in Table 3 below for analysis of the peptide solutions,

and chromatograms were obtained.

[HPLC analysis conditions]

Column: XSelect CSH C18, 5 μm, 4.6 × 250 mm, by Waters Co.
Guard column: XSelect CSH C18, 5 μm, 4.6 × 20 mm Guard Cartridge by Waters Co.
Detection wavelength: 220 nm
Mobile phase A: 0.1% formic acid aqueous solution
mobile phase B: 0.1% acetonitrile formate solution
Measuring time: 30 minutes
Measuring sample injection rate: 50 μL
Flow rate: 1.0 mL/min
Column temperature: 40°C
Mobile phase delivery: The mixing ratio of mobile phase A and mobile phase B was varied as shown in Table 3 for linear concentration gradient control.

[Table 3]

Table 3

| Time after sample injection (min) | Mobile phase A (vol%) | Mobile phase B (vol%) |
|---|---|---|
| 0 - 30.0 | 90 → 75 | 10 → 25 |
| 30.0 - 30.5 | 75 → 0 | 25 → 100 |
| 30.5 - 36.5 | 0 | 100 |
| 36.5 - 37.0 | 0 → 90 | 100 → 10 |
| 37.0 - 49.5 | 90 | 10 |

The peak area value for the peptide in the chromatogram was calculated, and the survival rate (survival rate before correction for water) was calculated using formula (2). The survival rate after correction for water was also calculated from the survival rate before correction for water using formula (3) to take into account the permeability of the container.
[Mathematical Formula 2]

$$\text{Survival rate before correction for water (\%)} = \frac{\text{Peptide peak area of stored sample}}{\text{peptide peak area of standard sample}} \times 100 \qquad (2)$$

[Mathematical Formula 3]

$$\text{Survival rate after correction for water (\%)} = \frac{\text{Survival rate before correction for water (\%)} \times (100\% - \text{vessel moisture permeability (\%)})}{100} \qquad (3)$$

Table 4 shows the survival rate after correction for water for each peptide among the stored samples.

[Table 4]

Table 4

| Peptide name | 40°C 4 week | 80°C 8 week |
|---|---|---|
| Peptide 1 | 91. 1% | 81.5% |
| Peptide 2 | 91.9% | 83.7% |
| Peptide 3 | 92.9% | 85.4% |
| Peptide 4 | 95.4% | 88.4% |
| Peptide 5 | 94.4% | 85.4% |
| Peptide 6 | 94.0% | 87.5% |
| Peptide 7 | 91.8% | 83.5% |
| Peptide 8 | 95.1% | 84.1% |
| Peptide 9 | 92.4% | 83.3% |
| PACAP27 | 37.4% | 21.3% |

All of the peptides exhibited high aqueous solution stability at 4 weeks and 8 weeks at 40°C, compared to PACAP27.

[Measuring sample preparation 2]

Peptide solutions prepared by the same method as [Measuring sample preparation 1] for the peptides synthesized in Example 2 (peptides 13 to 16) were incubated for 2 weeks and 8 weeks in a thermostatic bath at 40°C to obtain stored samples. A simultaneously prepared sample among the peptide solutions that was not stored was used as a standard sample (initial sample). The standard sample and the stored samples were stored at -30°C until sample analysis.

[HPLC analysis conditions]

Measurement was carried out under the same conditions as in [Measuring sample preparation 1], changing only the measuring time and mobile phase delivery conditions as shown below.

Measuring time: 20 minutes
Mobile phase delivery: The mixing ratio of mobile phase A and mobile phase B was varied as shown in Table 5 for linear concentration gradient control.

[Table 5]

Table 5

| Time after sample injection (min)) | Mobile phase A (vol%) | Mobile phase B (vol%) |
|---|---|---|
| 0 - 20.0 | 85 → 7 5 | 15 → 25 |
| 20.0 - 20.1 | 7 5 → 0 | 25 → 100 |
| 20.1 - 24.5 | 0 | 100 |
| 24.5 - 25.0 | 0 → 85 | 100 → 15 |
| 25.0 - 40.5 | 85 | 15 |

Table 6 shows the survival rate after correction for water for each peptide among the stored samples.

[Table 6]

Table 6

| Peptide name | 40°C 2 week | 40°C 8 week |
|---|---|---|
| Peptide 13 | 96.2% | 84.6% |
| Peptide 14 | 96. 3% | 84.5% |
| Peptide 15 | 95.6% | 81.3% |
| Peptide 16 | 94.4% | 80.3% |
| PACAP27 | 64.3% | 23.7% |

Even peptides with different the stereochemistry of the amino acid residues substituted with tetrazole and different lengths between the tetrazole and the peptide main chain exhibited higher stability than PACAP27.

[Measuring sample preparation 3]

Peptide solutions prepared by the same method as [Measuring sample preparation 1] for the peptides synthesized in Example 2 (peptides 17 to 21) were incubated for 4 weeks in a thermostatic bath at 40°C to obtain stored samples. A simultaneously prepared sample among the peptide solutions that was not stored was used as a standard sample (initial sample). The standard sample and the stored samples were stored at -30°C until sample analysis.

[HPLC analysis conditions]

Measurement was carried out under the same conditions as in [Measuring sample preparation 1], changing only the measuring time and mobile phase delivery conditions as shown below.

Measuring time: 20 minutes
Mobile phase delivery: The mixing ratio of mobile phase A and mobile phase B was varied as shown in Table 7 for linear concentration gradient control.

[Table 7]

Table 7

| Time after sample injection (min) | Mobile phase A (vol%) | Mobile phase B (vol%) |
|---|---|---|
| 0 - 20. 0 | 85 → 75 | 15 → 25 |
| 20.0 - 20.1 | 75 → 0 | 25 → 100 |
| 20.1 - 24.5 | 0 | 100 |
| 24.5 - 25.0 | 0 → 85 | 100 → 15 |
| 25.0 - 40.5 | 85 | 15 |

Table 8 shows the survival rate after correction for water for each peptide among the stored samples.

[Table 8]

Table 8

| Peptide name | 40°C 4 week |
|---|---|
| Peptide 17 | 59.2% |
| Peptide 18 | 65.1% |

(continued)

| Peptide name | 40°C 4 week |
|---|---|
| Peptide 19 | 68.7% |
| Peptide 20 | 69.5% |
| Peptide 21 | 69. 0% |
| PACAP27 | 23.9% |

Peptides with different acyl groups substituting at the N-terminus exhibited higher stability than PACAP27.

[Measuring sample preparation 4]

A peptide solution prepared by the same method as [Measuring sample preparation 1] for peptide 22 synthesized in Example 2 was incubated for 1 week and 4 weeks in a thermostatic bath at 40°C to obtain stored samples. A simultaneously prepared sample among the peptide solutions that was not stored was used as a standard sample (initial sample). The standard sample and the stored samples were stored at -30°C until sample analysis.

[HPLC analysis conditions]

Measurement was carried out under the same conditions as in [Measuring sample preparation 1], changing only the measuring time and mobile phase delivery conditions as shown below.

Measuring time: 20 minutes
Mobile phase delivery: The mixing ratio of mobile phase A and mobile phase B was varied as shown in Table 9 for linear concentration gradient control.

[Table 9]

Table 9

| Time after sample injection (min) | Mobile phase A (vol%) | Mobile phase B (vol%) |
|---|---|---|
| 0 - 20.0 | 85 → 75 | 15 → 25 |
| 20.0 - 20.1 | 75 → 0 | 25 → 100 |
| 20.1 - 24.5 | 0 | 100 |
| 24.5 - 25.0 | 0 → 85 | 100 → 15 |
| 25.0 - 40.5 | 85 | 15 |

Table 10 shows the survival rate after correction for water for each peptide among the stored samples.

[Table 10]

Table 10

| Peptide name | 40°C 1 week | 40°C 4 week |
|---|---|---|
| Peptide 22 | 89.7% | 79.7% |
| PACAP27 | 74. 9% | 33.0% |

Peptides having substitution of the tetrazole-substituted aspartic acid with oxadiazolone-substituted aspartic acid at position 3 and position 8 exhibited higher stability than PACAP27.

[Measuring sample preparation 5]

Peptides 23 to 30 synthesized in Example 2 were weighed and dissolved in Tris buffer (pH 7.0) to prepare 1.0 mM peptide solutions. The solution was filtered with a Chromatdisk (product of Merck Millipore, Millex-GV, 0.22 μm). The filtrate was diluted to 100 μM with Tris buffer (pH 7.0) and dispensed into a tube (Protein LoBind Tube by Eppendorf Co.). The prepared peptide solutions were incubated for 4 weeks or 8 weeks in a 40°C thermostatic bath, and for one week or 2 weeks in a thermostatic bath at 60°C, to obtain stored samples. A simultaneously prepared sample among the peptide solutions that was not stored was used as a standard sample (initial sample). The standard sample was stored at -30°C and the stored samples were stored at -30°C, until sample analysis.

[HPLC analysis conditions 1]

Measurement was carried out under the same conditions as in [Measuring sample preparation 1], changing only the measuring time and mobile phase delivery conditions as shown below.

Measuring time: 20 minutes
Mobile phase delivery: The mixing ratio of mobile phase A and mobile phase B was varied as shown in Table 11 for linear concentration gradient control.

[Table 11]

Table 11

| Time after sample injection (min) | Mobile phase A (vol%) | Mobile phase B (vol%) |
|---|---|---|
| 0 - 20.0 | 85 → 75 | 15 → 25 |
| 20.0 - 20.1 | 75 → 0 | 25 → 100 |
| 20.1 - 24.5 | 0 | 100 |
| 24.5 - 25.0 | 0 → 85 | 100 → 15 |
| 25.0 - 40.5 | 85 | 15 |

The following gradient time was used only for peptide 30.

[Table 12]

Table 12

| Time after sample injection (min) | Mobile phase A (vol%) | Mobile phase B (vol%) |
|---|---|---|
| 0 - 20.0 | 82.5 → 72.5 | 17.5 → 27.5 |
| 20.0 - 20.1 | 72.5 → 0 | 27.5 → 100 |
| 20.1 - 24.5 | 0 | 100 |
| 2 4.5 - 25.0 | 0 → 82.5 | 100 → 17.5 |
| 25.0 - 40.0 | 82.5 | 17.5 |

Table 13 shows the survival rate after correction for water for each peptide among the stored samples.

[Table 13]

Table 13

| Peptide name | 60°C 1 week | 60°C 2 week | 40°C 4 week | 40°C 8 week |
|---|---|---|---|---|
| Peptide 23 | 96.4% | 91.3% | 87.7% | 89.1% |
| Peptide 24 | 99.5% | 91.1% | 90.5% | 83.5% |
| Peptide 25 | 97.3% | 93.1% | 91.3% | 85.1% |
| Peptide 26 | 97.9% | 94.4% | 93.2% | 89.7% |
| Peptide 27 | 97.3% | 95.5% | 90.9% | 84.3% |
| Peptide 28 | 98.6% | 90.9% | 94.1% | 89.8% |
| Peptide 29 | 96.3% | 91.9% | 86.1% | 77.4% |
| Peptide 30 | 96.7% | 93.7% | 91.5% | 83.4% |
| PACAP27 | 26.4% | 14.6% | 37.9% | 15.6% |

Example 4: Stability test 2

N-terminal acylated peptides were prepared for peptides selected from the group consisting of peptides 1 to 16. Peptides without N-terminal acylated modification were prepared for peptides selected from the group consisting of peptides 17 to 30. The N-terminal modified peptides and the N-terminal non-modified peptides were each dissolved in Tris buffer (pH 7.0) to prepare 1.0 mM peptide solutions. The solution was filtered with a Chromatdisk (product of Merck Millipore, Millex-GV, 0.22 $\mu$m). The filtrate was diluted to 100 $\mu$M with Tris buffer (pH 7.0) and dispensed into a tube (Protein LoBind Tube by Eppendorf Co.). The prepared peptide solutions were incubated for 1 week or 2 weeks in a 60°C thermostatic bath to obtain stored samples. A simultaneously prepared sample among the peptide solutions that was not stored was used as a standard sample (initial sample). The standard sample and the stored samples were stored at -30°C until sample analysis.

The stored sample was analyzed under appropriate UPLC analysis conditions, and the survival rate after correction for water for the peptide in the stored sample was calculated in the same manner as Example 3. The N-terminal-modified, and especially N-terminal-acylated, and further acetylated peptides can be confirmed to have improved stability.

Reference Example: Stability test 3

The peptides prepared for Reference Examples 1 to 6 were weighed and dissolved in Tris buffer (pH 7.0) to prepare 1.0 mM peptide solutions. The solution was filtered with a Chromatdisk (product of Merck Millipore, Millex-GV, 0.22 $\mu$m). The filtrate was diluted to 100 $\mu$M with Tris buffer (pH 7.0) and dispensed into a tube (Protein LoBind Tube by Eppendorf Co.). The prepared peptide solutions were incubated for 1 week or 2 weeks in a 60°C thermostatic bath to obtain stored samples. A simultaneously prepared sample among the peptide solutions that was not stored was used as a standard sample (initial sample). The standard sample and the stored samples were stored at -30°C until sample analysis.

Each stored sample was measured under the following HPLC analysis conditions, and the survival rate after correction for water for each peptide among the stored samples was calculated in the same manner as Example 3. The results are shown in Table 15.

[HPLC analysis conditions]

Column: XSelect CSH C18 by Waters Co., 5 $\mu$m, 4.6 × 250 mm
Guard column: XSelect CSH C18 by Waters Co., 5 $\mu$m, 4.6 × 20 mm Guard Cartridge
Detection wavelength: 220 nm
Mobile phase A: 0.1% formic acid aqueous solution
Mobile phase B: 0.1% acetonitrile formate solution
Measuring time: 20 minutes
Measuring sample injection rate: 50 $\mu$L

Flow rate: 1.0 mL/min
Column temperature: 40°C
Sample cooler: 25°C
Mobile phase delivery: The mixing ratio of mobile phase A and mobile phase B was varied as shown in Table 14 for linear concentration gradient control.

[Table 14]

Table 14

| Time after sample injection (min)) | Mobile phase A (vol%) | Mobile phase B (vol%) |
|---|---|---|
| 0 - 20.0 | 85 → 75 | 15 → 25 |
| 20.0 - 20.1 | 75 → 0 | 25 → 100 |
| 20.1 - 24.5 | 0 | 100 |
| 24.5 - 25.0 | 0 → 85 | 100 → 15 |
| 25.0 - 40.5 | 85 | 15 |

Table 15 shows the survival rate after correction for water for each peptide among the stored samples.

[Table 15]

Table 15

| Peptide | 60°C 1 week preservation | 60°C 2 weeks preservation |
|---|---|---|
| Reference Example 1 | 26.6% | 15.8% |
| Reference Example 2 | 28.7% | 17.2% |
| Reference Example 3 | 91.3% | 82.4% |
| Reference Example 4 | 91.7% | 84.8% |
| Reference Example 5 | 98.1% | 92.3% |
| Reference Example 6 | 98.4% | 92.8% |

Reference Example 1 (PACAP) had very poor stability, at 26.6% after storage for 1 week at 60°C and 15.8% after storage for 2 weeks, while Reference Example 2, which had the N-terminus of PACAP acetylated, exhibited only equivalent stability. Reference Examples 3 and 4, on the other hand, which had the carboxyl groups of the aspartic acids at position 3 and position 8 replaced with tetrazole (Reference Example 3 also having the methionine at position 17 replaced by norleucine (indicated as "Nl" in the sequence) and Reference Example 4 further having the methionine at position 17 replaced by leucine), had drastically increased stability, at 91.3% and 91.7% after storage for 1 week at 60°C, and 82.4% and 84.8% after storage for 2 weeks. In Reference Examples 5 and 6, wherein the N-termini of Reference Examples 3 and 4 were further acetylated, stability was further improved, with 98.1% and 98.4% after storage for 1 week at 60°C, and 92.9% and 92.8% after storage for 2 weeks. An accelerated test for 1 week and 2 weeks at 60°C corresponds respectively to approximately 1 year and 2 years at room temperature (25°C). Therefore, Reference Examples 5 and 6 are expected to be stable (survival rate of 90% or higher) for 2 years at room temperature.

Example 5: cAMP assay of PACAP27 and its modified peptide

[Cell culturing]

CHO-K1 cells (PAC1 or VPAC1 receptor high-expressing cell line: purchased from DiscoveRx Co.) that had been mitomycin-treated, and frozen cells were prepared with Cell plating reagent (product of DiscoveRx) at $1.35 \times 10^4$

cells/100 μl/well, and then seeded in a 96-well culture plate. The cells were cultured for 18 to 24 hours at 37°C in a 5% $CO_2$ incubator, adhering the cells to the plate.

[Reagent preparation]

Peptides 1 to 30 synthesized in Example 2, and powder of Reference Example 1 (PACAP) were dissolved in water to 0.1 mM, and then diluted to 20 μM with Cell assay buffer (DiscoveRx) (containing 0.5 mM IBMX and 0.001% BSA). A 5-fold dilution series was then prepared with the same Cell assay buffer and used in an assay.

[cAMP assay]

The cAMP assay was carried out using a Hit Hunter cAMP Assay for Biologics kit (product of DiscoveRx, Cat. No. 90-0075LM25), according to the included kit instructions. The cAMP antibody solution and the solutions of peptides 1 to 30 diluted to different concentrations were combined to prepare peptide-cAMP antibody liquid mixtures. Next, the culture medium was removed from the CHO-K1 cell culture plate, washing was performed with PBS, and the peptide-cAMP antibody liquid mixtures were added to the cells and incubated for 30 minutes under a 5% $CO_2$ atmosphere at 37°C. A working detection solution was then added and the culture plate was shielded from light with aluminum foil and incubated at 25°C for 1 hour. After incubation, Solution A was added and the culture plate was shielded from light with aluminum foil and then incubated at 25°C for 3 hours. Finally, the chemiluminescence signal was detected using a GloMax detector (Promega) under conditions with luminescence and an integration time of 1 sec. The obtained value for the Relative Luminescence Unit (RLU) was analyzed with GraphPad Prism Ver 6.05 (product of Graph Pad) and the $EC_{50}$ value was calculated for each peptide.

[Table 16]

Table 16

| Peptide name | N-terminus | C-terminus | PAC1 | VPAC1 |
|---|---|---|---|---|
| Reference Example 1 (PACAP27) | H | NH2 | 0.052 nM | 0.097 nM |
| Peptide 1 | H | NH2 | 1.220 nM | 0.097 nM |
| Pept ide 2 | H | NH2 | 0.610 nM | 0.085 nM |
| Peptide 3 | H | NH2 | 0.689 nM | 0.111 nM |
| Peptide 4 | H | NH2 | 2.360 nM | 0.121 nM |
| Peptide 5 | H | NH2 | 0.695 nM | 0.078 nM |
| Peptide 6 | H | NH2 | 1.170 nM | 0.110 nM |
| Peptide 7 | H | NH2 | 0.319 nM | 0.096 nM |
| Peptide 8 | H | NH2 | 0.679 nM | 0.045 nM |
| Peptide 9 | H | NH2 | 0.287 nM | 0.079 nM |
| Peptide 10 | H | NH2 | 0.447 nM | 0.057 nM |
| Peptide 11 | H | NH2 | 0.911 nM | 0.080 nM |
| Peptide 12 | M | NH2 | 4.280 nM | 0.651 nM |
| Peptide 13 | H | NH2 | 0.738 nM | 0.082 nM |
| Peptide 14 | H | NH2 | 0.857 nM | 0.107 nM |
| Peptide 15 | H | NH2 | 0.079 nM | 0.243 nM |
| Peptide 16 | H | NH2 | 1.620 nM | 0.142 nM |
| Peptide 17 | $CH_3CH_2CO$ | NH2 | 0.398 nM | 0.071 nM |

(continued)

| Peptide name | N-terminus | C-terminus | PAC1 | VPAC1 |
|---|---|---|---|---|
| Peptide 18 | $CH_3CH_2CH_2CO$ | NH2 | 0.189 nM | 0.069 nM |
| Peptide 19 | $(CH_3)_2CHCO$ | NH2 | 0.256 nM | 0.069 nM |
| Peptide 20 | $CH_3CH_2CH_2CH_2CO$ | NH2 | 0.574 nM | 0. 131 nM |
| Peptide 21 | $(CH_3)_2CHCH_2CO$ | NH2 | 1.130 nM | 0.141 nM |
| Peptide 22 | Ac ($CH_3C0$) | NH2 | 0.376 nM | 0.077 nM |
| Peptide 23 | Ac | NH2 | 1.150 nM | 0.047 nM |
| Peptide 24 | Ac | NH2 | 1.260 nM | 0.042 nM |
| Peptide 25 | Ac | NH2 | 0.693 nM | 0.041 nM |
| Peptide 26 | Ac | NH2 | 0.209 nM | 0.068 nM |
| Peptide 27 | Ac | NH2 | 2.530 nM | 0.111 nM |
| Peptide 28 | Ac | NH2 | 2.120 nM | 0.072 nM |
| Peptide 29 | Ac | NH2 | 3.280 nM | 0.123 nM |
| Peptide 30 | Ac | NH2 | 2.290 nM | 0.113 nM |

To summarize the results described above, the peptides of the invention (peptides 1 to 30) have extremely improved stability in aqueous solution compared to PACAP, while also maintaining physiological activity equivalent to that of PACAP. In particular, because they have storage life exceeding 2 years at room temperature, they allow liquid formulations to be developed as products such as vials, ampules and eye drops.

Formulation Examples

A medicine containing a peptide of the invention as an active ingredient can be produced by the following formulation. The medicine of the present invention will now be further illustrated by formulation examples, with the understanding that the invention is not limited to the formulation examples.

1. Capsules

| | |
|---|---|
| (1) Peptide 5 | 40 mg |
| (2) Lactose | 70 mg |
| (3) Microcrystalline cellulose | 9 mg |
| (4) Magnesium stearate | 1 mg |
| 1 capsule: | 120 mg |

After mixing the total amounts of (1), (2) and (3) and 1/2 of (4), the mixture is granulated. The remaining amount of (4) is added and the entire mixture is encapsulated into gelatin capsules.

2. Tablets

| | |
|---|---|
| (1) Peptide 6 | 40 mg |
| (2) Lactose | 58 mg |
| (3) Corn starch | 18 mg |
| (4) Microcrystalline cellulose | 3.5 mg |

(continued)

| | |
|---|---|
| (5) Magnesium stearate | 0.5 mg |
| 1 tablet | 120 mg |

After mixing the total amounts of (1), (2) and (3), 2/3 of (4) and 1/2 of (5), the mixture is granulated. The remaining amounts of (4) and (5) are added to the granules, which are then pressure molded into tablets.

3. Vitreous injection

| | | |
|---|---|---|
| | In 1 ml: | |
| (1) | Peptide 5 | 40 mg |
| (2) | Refined sucrose | 50 mg |
| (3) | Sodium chloride | 2.34 mg |
| (4) | Polysorbate 80 | q.s. |
| (5) | Disodium hydrogenphosphate | q.s |
| (6) | Sodium dihydrogenphosphate | q.s |
| (7) | Sterilized purified water | q.s. |

Components (1) to (6) are dissolved in the sterilized purified water (7) to prepare a vitreous injection.

4. Ophthalmic drug

| | | |
|---|---|---|
| | In 100 mL: | |
| (1) | Peptide 6 | 100 mg |
| (2) | Trometamol | 300 mg |
| (3) | Sodium chloride | 900 mg |
| (4) | Benzalkonium chloride | q.s. |
| (5) | Sterilized purified water | q.s. |

Components (1) to (4) were dissolved in the sterilized purified water (5) and the pH was adjusted to prepare eye drops.

## Claims

**1.** A peptide consisting of the sequence represented by H-$X_1$-D-G-$X_2$-F-$X_3$-D-$X_4$-Y-$X_5$-R-Y-R-K-$X_6$-$X_7$-A-V-K-K-Y-L-A-A-V-L (SEQ ID NO: 3)
{wherein

$X_1$ is a neutral amino acid,
$X_2$ is a neutral amino acid,
$X_3$ is a neutral amino acid,
$X_4$ is alanine, serine, Ai or Cn,
$X_5$ is alanine, serine, Ai or Cn,
$X_6$ is glutamine or alanine and
$X_7$ is non-polar amino acid},
with the aspartic acids at position 3 and position 8 each independently replaced by a residue selected from the group consisting of tetrazole-substituted aspartic acid (Tz), tetrazole-substituted glutamic acid (egTz), tetrazole-substituted homoglutamic acid (nvTz), sulfoxy-substituted aspartic acid (cya) and oxadiazolone-substituted aspartic acid (SOxa), or its modified sequence, wherein the peptide has affinity for PAC1R and VPAC1R, and the modified sequence is the sequence listed as SEQ ID NO: 3, wherein:

(A) 1 to 10 amino acids are added at the N-terminal end or C-terminal end, or
(B) one sequence selected from the group consisting of the following:

GKRYKQRVKNK (SEQ ID NO: 43);
GKRYKQRVKN (SEQ ID NO: 44);
GKRYKQRVK (SEQ ID NO: 45);
GKRYKQRV (SEQ ID NO: 46);
GKRYKQR (SEQ ID NO: 47);
GKRYKQ (SEQ ID NO: 48);
GKRYK (SEQ ID NO: 49);
GKRY (SEQ ID NO: 50)
GKR;
GRR;
GK; and
GR
G;
is added to the C-terminus of the peptide, or

(C) one or two, preferably one amino acid is deleted at the N-terminal or C-terminal end.

**2.** The peptide according to claim 1, wherein $X_1$ is serine or alanine, and/or

wherein $X_2$ is isoleucine or alanine, and/or
wherein $X_3$ is alanine or threonine, and/or
wherein $X_7$ is methionine, leucine, norleucine or alanine.

**3.** The peptide according to any one of claims 1 to 2, wherein in cases where the amino acids at position 3 and position 8 are tetrazole-substituted aspartic acid (Tz), tetrazole-substituted glutamic acid (egTz) or tetrazole-substituted homoglutamic acid (nvTz), the amino acids are the L-forms, D-forms or racemic forms.

**4.** The peptide according to any one of claims 1 to 3, wherein the N-terminus of the peptide is modified with a protecting group, wherein optionally the protecting group is an acyl group.

**5.** The peptide according to any one of claims 1 to 4, wherein the C-terminus of the peptide is modified with a protecting group, wherein optionally the protecting group is an amide group or ester group.

**6.** The peptide according to any one of claims 1(A) to 5, wherein the modified sequence is the sequence with 1 to 3 amino acids added to the N-terminus of the sequence.

**7.** A neuroprotective agent containing the peptide according to any one of claims 1 to 6.

**8.** A lacrimal secretagogue containing the peptide according to any one of claims 1 to 6.

**9.** An anti-inflammatory agent containing the peptide according to any one of claims 1 to 6.

**10.** A vascular endothelial function ameliorator containing the peptide according to any one of claims 1 to 6.

**11.** A corneal epithelial or corneal endothelial damage treatment agent containing the peptide according to any one of claims 1 to 6.

**12.** A dry-eye treatment agent containing the peptide according to any one of claims 1 to 6.

**13.** A pharmaceutical composition containing the peptide according to any one of claims 1 to 6.

## Patentansprüche

**1.** Peptid bestehend aus der Sequenz, dargestellt durch H-$X_1$-D-G-$X_2$-F-$X_3$-D-$X_4$-Y-$X_5$-R-Y-R-K-$X_6$-$X_7$-A-V-K-K-Y-L-A-A-V-L (SEQ ID NO: 3)
{wobei

$X_1$ eine neutrale Aminosäure ist,
$X_2$ eine neutrale Aminosäure ist,
$X_3$ eine neutrale Aminosäure ist,
$X_4$ Alanin, Serin, Ai oder Cn ist,
$X_5$ Alanin, Serin, Ai oder Cn ist,
$X_6$ Glutamin oder Alanin ist und
$X_7$ eine unpolare Aminosäure ist},
wobei die Asparaginsäuren an Position 3 und Position 8 jeweils unabhängig voneinander durch einen Rest ersetzt sind, der ausgewählt ist aus der Gruppe bestehend aus Tetrazol-substituierter Asparaginsäure (Tz), Tetrazol-substituierter Glutaminsäure (egTz), Tetrazol-substituierter Homoglutaminsäure (nvTz), Sulfoxysubstituierter Asparaginsäure (cya) und Oxadiazolonsubstituierter Asparaginsäure (5Oxa), oder ihre modifizierten Sequenz, wobei das Peptid Affinität für PAC1R und VPAC1R hat und die modifizierte Sequenz die als SEQ ID NO: 3 gelistete Sequenz ist, wobei:

(A) 1 bis 10 Aminosäuren am N-terminalen Ende oder C-terminalen Ende hinzugefügt werden, oder
(B) eine Sequenz ausgewählt aus der Gruppe bestehend aus den folgenden:

GKRYKQRVKNK (SEQ ID NO: 43);
GKRYKQRVKN (SEQ ID NO: 44);
GKRYKQRVK (SEQ ID NO: 45);
GKRYKQRV (SEQ ID NO: 46);
GKRYKQR (SEQ ID NO: 47);
GKRYKQ (SEQ ID NO: 48);
GKRYK (SEQ ID NO: 49);
GKRY (SEQ ID NO: 50)
GKR;
GRR;
GK; und
GR
G;
an den C-Terminus des Peptids angefügt ist, oder

(C) eine oder zwei, vorzugsweise eine Aminosäure am N-terminalen oder C-terminalen Ende entfernt ist.

**2.** Peptid gemäß Anspruch 1, wobei $X_1$ Serin oder Alanin ist, und/oder

wobei $X_2$ Isoleucin oder Alanin ist, und/oder
wobei $X_3$ Alanin oder Threonin ist, und/oder
wobei $X_7$ Methionin, Leucin, Norleucin oder Alanin ist.

**3.** Peptid gemäß irgendeinem der Ansprüche 1 bis 2, wobei in den Fällen, in denen die Aminosäuren an Position 3 und Position 8 Tetrazol-substituierte Asparaginsäure (Tz), Tetrazol-substituierte Glutaminsäure (egTz) oder Tetrazol-substituierte Homoglutaminsäure (nvTz) sind, die Aminosäuren die L-Formen, D-Formen oder racemischen Formen sind.

**4.** Peptid gemäß irgendeinem der Ansprüche 1 bis 3, wobei der N-Terminus des Peptids mit einer Schutzgruppe modifiziert ist, wobei optional die Schutzgruppe eine Acylgruppe ist.

**5.** Peptid gemäß irgendeinem der Ansprüche 1 bis 4, wobei der C-Terminus des Peptids mit einer Schutzgruppe modifiziert ist, wobei optional die Schutzgruppe eine Amidgruppe oder Estergruppe ist.

**6.** Peptid gemäß irgendeinem der Ansprüche 1(A) bis 5, wobei die modifizierte Sequenz die Sequenz ist, bei der 1 bis 3 Aminosäuren an den N-Terminus der Sequenz angefügt sind.

**7.** Neuroprotektives Mittel, enthaltend das Peptid gemäß irgendeinem der Ansprüche 1 bis 6.

8. Lakrimales Sekretagogum, enthaltend das Peptid gemäß irgendeinem der Ansprüche 1 bis 6.

9. Entzündungshemmendes Mittel, enthaltend das Peptid gemäß irgendeinem der Ansprüche 1 bis 6.

10. Verbesserer der vaskulären Endothelfunktion, enthaltend das Peptid gemäß irgendeinem der Ansprüche 1 bis 6.

11. Mittel zur Behandlung von Hornhautepithel- oder Hornhautendothelschäden, enthaltend das Peptid gemäß irgendeinem der Ansprüche 1 bis 6.

12. Mittel zur Behandlung von trockenen Augen, enthaltend das Peptid gemäß irgendeinem der Ansprüche 1 bis 6.

13. Pharmazeutische Zusammensetzung, enthaltend das Peptid gemäß irgendeinem der Ansprüche 1 bis 6.

## Revendications

1. Peptide constitué de la séquence représentée par H-$X_1$-D-G-$X_2$-F-$X_3$-D-$X_4$-Y-$X_5$-R-Y-R-K-$X_6$-$X_7$-A-V-K-K-Y-L-A-A-V-L (SEQ ID NO : 3)
{dans lequel

$X_1$ est un acide aminé neutre,
$X_2$ est un acide aminé neutre,
$X_3$ est un acide aminé neutre,
$X_4$ est l'alanine, la sérine, Ai ou Cn,
$X_5$ est l'alanine, la sérine, Ai ou Cn,
$X_6$ est la glutamine ou l'alanine et
$X_7$ est un acide aminé non polaire},
avec les acides aspartiques en position 3 et position 8 remplacés chacun indépendamment par un résidu choisi dans le groupe constitué d'acide aspartique substitué par le tétrazole (Tz), d'acide glutamique substitué par le tétrazole (egTz), d'acide homoglutamique substitué par le tétrazole (nvTz), d'acide aspartique substitué par le sulfoxy (cya) et d'acide aspartique substitué par l'oxadiazolone (5Oxa), ou sa séquence modifiée, dans lequel le peptide présente une affinité pour PAC1R et VPAC1R, et la séquence modifiée est la séquence listée selon la SEQ ID NO : 3, dans lequel :

(A) 1 à 10 acides aminés sont ajoutés à l'extrémité N-terminale ou à l'extrémité C-terminale, ou
(B) une séquence choisie dans le groupe constitué des séquences suivantes :

GKRYKQRVKNK (SEQ ID NO : 43) ;
GKRYKQRVKN (SEQ ID NO : 44) ;
GKRYKQRVK (SEQ ID NO : 45) ;
GKRYKQRV (SEQ ID NO : 46) ;
GKRYKQR (SEQ ID NO : 47) ;
GKRYKQ (SEQ ID NO : 48) ;
GKRYK (SEQ ID NO : 49) ;
GKRY (SEQ ID NO : 50)
GKR ;
GRR ;
GK ; et
GR
G ;

est ajoutée au C-terminus du peptide, ou
(C) un, ou deux, de préférence un acide aminé est supprimé à l'extrémité N-terminale ou C-terminale.

2. Peptide selon la revendication 1, dans lequel $X_1$ est la sérine ou l'alanine, et/ou

dans lequel $X_2$ est l'isoleucine ou l'alanine, et/ou
dans lequel $X_3$ est l'alanine ou la thréonine, et/ou

dans lequel $X_7$ est la méthionine, la leucine, la norleucine ou l'alanine.

**3.** Peptide selon l'une quelconque des revendications 1 à 2, dans lequel dans les cas où les acides aminés en position 3 et position 8 sont l'acide aspartique substitué par le tétrazole (Tz), l'acide glutamique substitué par le tétrazole (egTz) ou l'acide homoglutamique substitué par le tétrazole (nvTz), les acides aminés sont des formes L, des formes D ou des formes racémiques.

**4.** Peptide selon l'une quelconque des revendications 1 à 3, dans lequel le N-terminus du peptide est modifié par un groupe protecteur, dans lequel en option le groupe protecteur est un groupe acyle.

**5.** Peptide selon l'une quelconque des revendications 1 à 4, dans lequel le C-terminus du peptide est modifié par un groupe protecteur, dans lequel en option le groupe protecteur est un groupe amide ou groupe ester.

**6.** Peptide selon l'une quelconque des revendications 1(A) à 5, dans lequel la séquence modifiée est la séquence avec 1 à 3 acides aminés ajoutés au N-terminus de la séquence.

**7.** Agent neuroprotecteur contenant le peptide selon l'une quelconque des revendications 1 à 6.

**8.** Sécrétagogue lacrymal contenant le peptide selon l'une quelconque des revendications 1 à 6.

**9.** Agent anti-inflammatoire contenant le peptide selon l'une quelconque des revendications 1 à 6.

**10.** Améliorateur de la fonction endothéliale vasculaire contenant le peptide selon l'une quelconque des revendications 1 à 6.

**11.** Agent de traitement des dommages épithéliaux cornéens ou endothéliaux cornéens contenant le peptide selon l'une quelconque des revendications 1 à 6.

**12.** Agent de traitement de la sécheresse oculaire contenant le peptide selon l'une quelconque des revendications 1 à 6.

**13.** Composition pharmaceutique contenant le peptide selon l'une quelconque des revendications 1 à 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014510101 A
- JP 2012232952 A
- JP 2009269818 A
- WO 2005102375 A
- WO 2017027848 A


### Non-patent literature cited in the description


- **S. BOURGAULT.** *Current Medicinal Chemistry,* 2009, vol. 16, 4462-4480
- **LOUISE DICKSON.** *Pharmacology & Therapeutics,* 2009, vol. 12, 294-316